# EUROPEAN PATENT SPECIFICATION

(11) **EP 1 395 284 B1**
(45) Date of publication and mention of the grant of the patent: **08.12.2010**
(21) Application number: 02730530.9
(22) Date of filing: 14.06.2002
(51) Int. Cl.: A61K 39/395

(54) **Non-depleting anti-CD4 antibody TRX1 and its uses**
Nicht-depletierender Antikörper TRX-1 gegen CD4 und dessen Verwendungen
Anticorps anti-CD4 TRX1 non-déplétant et utilisations associées

(30) Priority: 14.06.2001 GB 0114517; 20.09.2001 GB 0122724; 19.10.2001 US 345194 P; 18.04.2002 US 373470 P; 18.04.2002 US 373471 P
(43) Date of publication of application: 10.03.2004
(60) Divisional application: 08011443.2
(73) Proprietor: ISIS INNOVATION LIMITED, Summertown, Oxford OX2 7SG (GB); Cambridge Enterprise Limited, Cambridge CB3 0GT (GB); Tolerx, Inc., Cambridge, MA 02139 (US)
(72) Inventor: FREWIN, Mark, Oxford, Oxfordshire OX4 2JJ (GB); WALDMANN, Herman, Oxford, Oxfordshire OX2 7QY (GB); GORMAN, Scott, Witney, Oxfordshire OX8 8HL (GB); HALE, Geoff, Oxford, Oxfordshire OX3 0SJ (GB); RAO, Patricia, Acton, MA 01720 (US); KORNAGA, Tadeusz, Cambridge, MA 02139 (US); RINGLER, Douglas, Boston, MA 02116 (US); COBBOLD, Stephen, Stonesfield, Witney, Oxon OX8 8PS (GB); WINSOR-HINES, Dawn, Framingham, MA 01701 (US)
(74) Representative: Icely, Dominic Michael
(86) International application number: PCT/GB2002/002796
(87) International publication number: WO 2002/102853

(56) References cited:
- WO-A-90/15152
- WO-A-96/36359
- ISAACS J D ET AL: "Humanized anti-CD4 monoclonal antibody therapy of autoimmune and inflammatory disease." CLINICAL AND EXPERIMENTAL IMMUNOLOGY, vol. 110, no. 2, November 1997 (1997-11), pages 158-166, XP009007437 ISSN: 0009-9104
- STEPHENS LEIGH A ET AL: "Human CD4+CD25+ thymocytes and peripheral T cells have immune suppressive activity in vitro" EUROPEAN JOURNAL OF IMMUNOLOGY, WEINHEIM, DE, vol. 31, no. 4, April 2001 (2001-04), pages 1247-1254, XP002210570 ISSN: 0014-2980
- STUMBLES P ET AL: "ACTIVATION OF CD4+ T CELLS IN THE PRESENCE OF A NONDEPLETING MONOCLONAL ANTIBODY TO CD4 INDUCES A TH2-TYPE RESPONSE IN VITRO" JOURNAL OF EXPERIMENTAL MEDICINE, TOKYO, JP, vol. 182, no. 1, 1 July 1995 (1995-07-01), pages 5-13, XP002051825 ISSN: 0022-1007
- WINSOR-HINES ET AL: "Induction of immunological Tolerance/Hyporesponsiveness in baboons with a nondepleting CD4 antibody" THE JOURNAL OF IMMUNOLOGY, vol. 173, pages 4715-4723,
- NG ET AL: "Pharmacokinetics/Pharmacodynamics of Nondepleting anti-CD4 monoclonal antibody (TRX1) in healthy human volunteers" PHARMACEUTICAL RESEARCH, vol. 23, no. 1, 1 January 2006 (2006-01-01), pages 95-103,

## Description

The present invention is applicable to inhibiting, preventing or ameliorating an immune response against an antigen(s). Such inhibiting, preventing, or ameliorating an immune response against an antigen(s) includes inducing tolerance to the antigen(s). This invention also relates to tolerance induction and/or preventing or inhibiting T cell activation and proliferation and, more particularly, to inducing tolerance in a primate.

### BACKGROUND OF THE INVENTION

Tolerance to foreign antigen or tissue, or self antigen or tissue is a state whereby an otherwise normal, mature immune system is specifically unable to respond aggressively to that antigen/tissue which it therefore treats like a normal (non-diseased) body tissue/component, yet at the same time it can respond aggressively to foreign or diseased antigens/tissues to which it has not been specifically made tolerant by the natural process of self tolerance or by creating a tolerance-permissive environment *in vivo*. D2 (Isaacs et al, Clin. Exp. Immunology 1997, 110, pp 158-166) discloses a humanized non-depleting anti-CD4 Ab, but this showed severe first dose toxicity on administration.

### SUMMARY OF THE INVENTION

In accordance with one aspect of the present invention there is provided a humanized anti-CD4 non-depleting antibody comprising a Light Chain and a Heavy Chain, the Light Chain having the three Light Chain CDRs KASQSVDYDGDSYMN, VASNLES and QQSLQDPPT, and the Heavy Chain having the three Heavy Chain CDRs AYVIS, EIYPGSGSSYYNEKFKG and SGDGSRFVY, the antibody having an aglycosylated Fc portion.

In accordance with another aspect of the present invention there are provided uses for said antibodies.

In accordance with a further aspect of the invention there is provided a process for inducing tolerance in a primate by use of said antibodies by use of a dosing regimen that induces such tolerance in a primate.

### BRIEF DESCRIPTION OF THE DRAWING

Figure 1 shows the amino acid sequences of the heavy and light chains of the one example of TRX1 antibody, as well as the CDR and framework regions of the heavy and light chains.
Figure 2 shows the amino acid sequences of the heavy and light chains of one embodiment of the present TRX1 antibody, as well as the CDR and framework regions of the heavy and light chains.
Figure 3 shows the amino acid sequences of the heavy and light chains of another example of the TRX1 antibody, as well as the CDR and framework regions of the heavy and light chains.
Figure 4 shows the amino acid sequences of the heavy and light chains of a second embodiment of the present TRX1 antibody, as well as the CDR and framework regions of the heavy and light chains.
Figure 5 shows the immune response to antivenin for the first 68 days (i.e., the tolerization phase) of a study of groups of baboons which were given antivenin alone or in combination with TRX1 antibody.
Figure 6 shows the immune response to antivenin, subsequent to an antivenin and sheep red blood cell challenge, to groups of baboons which were given antivenin, alone or in combination with TRX1 antibody, 68 days before the challenge.
Figure 7 shows the immune response to antivenin, subsequent to each of three antivenin challenges, to groups of baboons which were given antivenin, alone or in combination with TRX1 antibody, 68 days before the first challenge.
Figure 8 is a chart depicting immune response to sheep red blood cells after TRX1 tolerization against antivenin.

### DETAILED DESCRIPTION OF THE INVENTION

In accordance with an aspect of the present invention, there is provided a process for tolerizing a primate against an antigen(s) by use of an antibody of the present invention hereinafter described. The Ab is administered in an amount and in accordance with a dosage regimen that is effective for inducing tolerance in a primate. The Ab is an Ab that when present in a primary mixed lymphocyte reaction (MLR) as compared to a mixed lymphocyte reaction in the absence of the Ab reduces the amount of cells that are positive for both CD4 and CD25 (CD4+CD25+cells) that result from the mixed lymphocyte reaction. In a preferred embodiment, the Ab is an Ab that reduces the amount of such CD4+CD25+cells by at least 40% and preferably by at least 60% and still more preferably by at least 70%.

Reference hereinafter to "compound" will be understood to be to the antibodies as the present invention.

In a preferred embodiment, such a compound produces a cell population that inhibits a primary mixed lymphocyte reaction (performed with cells that have not been previously exposed to the compound) by reducing the CD4+ CD25+ cells produced in the primary MLR. In a preferred embodiment, there is at least a 10% reduction and preferably at least a 20% reduction. Protocols for performing the hereinabove described mixed lymphocyte reactions are described in Example 5.

In addition, in a preferred embodiment, the compound is a compound that reduces the amount of CD4+ CD25+cells produced in a primary mixed lymphocyte reaction and generates a cell population in such primary mixed lymphocyte reaction which cell population inhibits a secondary mixed lymphocyte reaction. In a preferred embodiment, the cells are generated in a primary mixed lymphocyte reaction that is performed in the presence of the compound, which cells when added to the secondary mixed lymphocyte reaction preferably reduce the CD4+ CD25+ cells generated in such secondary mixed lymphocyte reaction, as compared to a secondary mixed lymphocyte reaction in the absence of the added cells by at least 20% more preferably by at least 35%, and more preferably by at least 50%.

An inhibition of either a primary or secondary MLR by such a cell population is evidenced by a reduction in the amount of CD4+ CD25+cells produced in the MLR as compared to the MLR performed in the absence of such a cell population.

Thus, in accordance with an aspect of the present invention, a primate is treated with a compound having the characteristics as hereinabove described (reduction of the amount of CD4+ CD25+cells produced in an in vitro primary MLR, with such primary MLR generating a cell population that reduces the amount of CD4+ CD25+cells produced in vitro in a primary and/or secondary MLR and that preferably effects such reduction in both a primary and secondary MLR).

In one embodiment, the cells generated in the primary MLR in the presence of the compound are cells which when added to a secondary MLR (as compared to a secondary MLR without the added cells) reduces and/or eliminates the generation of one or more of cytokines; in particular, one or more of IL-2, IL-4 and IL-12 in the secondary MLR. In general, such reduction of at least one of IL-2, IL-4 and IL-12 is at least 40%, preferably by at least 60%.

Thus, a preferred compound is one that generates cells in a primary MLR that in a secondary MLR, as compared to control, reduces production of CD₄⁺CD₂₅⁺ cells or one or more or all (preferably all) of IL-2, IL-4 and IL-12 and preferably reduces production of both such cells and such cytokines.

Herein disclosed is a screen or test in which, in vitro, T cells are exposed to a material(s) or compound(s) under conditions which stimulate and cause the T cells to proliferate, with such exposure being effected in the presence of a compound(s) that is to be tested for its ability to induce tolerance. The T cell proliferation test may be a mixed lymphocyte reaction or a test in which T cells are caused to proliferate by a non-antigen specific stimulation through the T cell receptor (TCR) or a component of the TCR complex, for example a CD3 component. Typically, an anti-CD3 monoclonal antibody is used to stimulate T cells and cause them to proliferate. In addition to stimulation through the TCR complex, co-stimulatory signals sometimes are provided by addition of antibodies which bind co-stimulatory molecules such as CD28. The T cells that have been caused to proliferate, in the presence and absence of the compound that is being tested, are examined to determine a subset of T cells that are CD4 positive (CD4⁺) and CD25 positive (CD25⁺) in order to determine whether the presence of the compound inhibited the production of such T cell subset.

The in vitro test that is used to test a compound(s) for tolerance-inducing activity is preferably a mixed lymphocyte reaction (MLR). Mixed lymphocyte reactions are generally known in the art, and a protocol therefor is described in Example 5.

The cell population produced in the initial test may then be tested in at least one further test in which T cells, in vitro are caused to proliferate in order to determine whether such cell population inhibits production of CD4⁺ CD25⁺ cells.

The at least one further test may be a mixed lymphocyte reaction (a primary or secondary mixed lymphocyte reaction) or a test in which T cells are caused to proliferate in response to a non-antigen specific stimulation through the T cell receptor (TCR) or a component of the TCR complex or with co-stimulation which mimics an antigen specific stimulation thorugh TCR.

A protocol for such a test is described in Example 5.

The screen or test may involve both the initial test to determine whether the compound in an in vitro T cell proliferation test, such as an MLR, reduces the production of CD4⁺ CD25⁺ cells and a subsequent test to determine whether cells produced in the first test inhibit the production of CD4⁺ CD25⁺ cells in a second T cell proliferation test, such as a primary and/or secondary MLR and, in particular, a secondary MLR and/or cytokine production in a secondary MLR.

The Ab is one that causes reduction of production of CD4⁺ CD25⁺ cells in the initial test and produces a cell population therein that reduces production of such T cell subset in a second test, as hereinabove described and/or that reduces cytokine production and, in particular, one or more of IL-2, IL-4 and IL-12 in a second test.

The Ab is a compound that when present in a T cell proliferation assay such as a primary mixed lymphocyte reaction (MLR) as compared to a mixed lymphocyte reaction in the absence of the compound reduces the amount of cells that are positive for both CD4 and CD25 (CD4+CD25+cells) that result from the mixed lymphocyte reaction. In a preferred embodiment, the compound is a compound that reduces the amount of such CD4+CD25+cells by at least 60% and still more preferably by at least 70%.

In addition, in a preferred embodiment, the selected compound is a compound that reduces the amount of CD4+ CD25+cells produced in a primary mixed lymphocyte reaction and generates a cell population in such primary mixed lymphocyte reaction which cell population inhibits a secondary mixed lymphocyte reaction. In a preferred embodiment, the cells are generated in a primary mixed lymphocyte reaction that is performed in the presence of the compound, which cells when added to the secondary mixed lymphocyte reaction preferably reduce the CD4+ CD25+ cells generated in such secondary mixed lymphocyte reaction, (as compared to a secondary mixed lymphocyte reaction in the absence of the added cells) by at least 20% more preferably by at least 35% and more preferably by at least 50%. In a preferred embodiment, such cells in the secondary MLR, as compared to a control, reduce the production of at least one of IL-2, IL-4 and IL-12 by at least 60%.

An inhibition of either a primary or secondary MLR by such a cell population is evidenced by a reduction in the amount of CD4+ CD25+cells produced in the MLR as compared to the MLR performed in the absence of such a cell population, and with respect to the secondary MLR, may be evidenced by reduction of CD4⁺CD25⁺ cells or reduced cytokine production and preferably by both.

As used herein, the term "tolerize" or "tolerant" with respect to an antigen means that, without requiring a therapeutic or effective level of an immunosuppressant, the primate does not produce an adverse immune response to the antigen over a period of time after treatment is stopped even when subsequently challenged with the antigen and/or when the antigen remains present in the primate, but is capable of providing an immune response against other antigens.

The antigen(s) as to which tolerance is induced may be a self antigen or a foreign antigen.

The foreign antigen may be one or more of the following types of antigens:
(i) a foreign antigen present on transplanted tissue or cells, including tissue or cells present in an organ wherein the transplant may be allogeneic or xenogeneic;
(ii) a therapeutic agent (which also includes therapeutic agents used for disease prevention) that produces an immune response in a primate, which immune response diminishes the ability of the agent to function as a therapeutic agent. Such agents include, but are not limited to, delivery vehicles, such as vectors used in gene therapy; active agents such as proteins delivered to the primate, such as recombinant proteins such as monoclonal antibodies, enzymes, clotting factors and some small molecule drugs, or proteins produced from an agent delivered to the primate, such as in gene therapy.

The foreign antigens against which tolerance is induced in accordance with the present invention are not foreign antigens as present in disease-causing bacteria, fungi, viruses, etc. that infect a host, i.e., the term foreign antigen does not include a foreign antigen as part of an organism that infects a primate and causes a disease or disorder.

In accordance with one aspect, a primate is treated to produces tolerance against an antigen(s) by treating the primate with the CD4 antibody in an amount and for a time that is effective for providing such tolerance, with the antibody being present in the primate at a time when such antigen is also present in the primate, with such treatment resulting in the primate being tolerant to the antigen. Such CD4 antibody has the hereinabove described characteristics when tested in vitro in an MLR (reduces the amount of CD4+ CD25+ cells produced in a primary MLR, with the cell population produced in the primary MLR when tested in vitro in at least one of a primary or a secondary MLR reduces the amount of CD4+ CD25+ cells produced therein).

The CD4 antibody is a monoclonal antibody. The antibody is a humanized Ab.

The CD4 antibody is administered to a primate in an amount and for a time effective to induce tolerance against a foreign or self-antigen and preferably a foreign antigen.

In accordance with a preferred embodiment, the CD4 antibody thereof is administered over a period of time in order to maintain in the primate appropriate levels of such antibody or fragment over a period of time that is sufficient to induce tolerance.

In general, the antibody is administered in an initial dose of at least about 40 mg, preferably at least about 50 mg and more preferably in an amount of at least about 70 mg.

In one preferred embodiment, the initial dose is at least 400 mg, preferably at least about 500 mg and in a particular embodiment in an amount of at least about 700 mg.

The initial dose may be administered in one or more doses over a twenty-four hour period and preferably in one dose over twenty-four hours.

As used herein in reference to a dosage amount a dose is the total amount of antibody administered over a twenty-four hour period, even if administered more than once in 24 hours.

In most cases, after the initial dose, the CD4 antibody is administered in one or more follow-up doses over several day(s), with each follow-up dose being administered in one or more doses in a twenty-four hour period. The follow-up dose(s) is generally provided in an amount to return serum levels of the antibody to those that were achieved by the initial dose.

In a preferred embodiment, the minimum follow-up dose or doses is in an amount that is at least equal to the amounts hereinabove described and may or may not be identical to the dose given as the original or initial dose. Thus, a follow-up dose is generally at least 40 mg, preferably at least 50 mg, and more preferably at least 70 mg. As hereinabove described, in one preferred embodiment, the follow-up dose(s) is at least 400 mg, preferably at least 500 mg, and in a particular embodiment at least 700 mg. In some cases, the follow-up dose or doses may be less than the minimum amount.

If there is more than one follow-up dose, each such additional follow-up dose over a 24-hour period may be the same or different than another follow-up dose.

The number of follow-up doses will vary, but in a preferred embodiment, there is generally at least one follow-up dose and in most cases there is no more than seven follow-up doses, i.e., the total number of doses generally does not exceed eight doses.

The total period over which the antibody is administered generally does not exceed four weeks and more preferably does not exceed three weeks. In many cases, tolerance can be achieved by using an initial dose and one or more follow-up doses over a period that does not exceed two weeks.

Although, in accordance with the present invention, initial tolerance to an antigen(s) can be achieved in a primate in a period of no more than four weeks, in some cases, periodic follow-up treatments with the antibody may be required in order to maintain tolerance.

As hereinabove described, at least one CD4 antibody is delivered in an amount that is at least sufficient to induce tolerance in a primate against an antigen(s) and in a preferred embodiment against a foreign antigen. The maximum amount is of course limited by safety considerations. In general, the daily dosage of antibody would be less than 6000 mg.

The number of follow-up doses and the spacing thereof will be determined, in part, by the half-life of the at least one CD4 antibody. Although the present invention is not to be limited thereby, it is believed that the CD4 antibody should be initially delivered in an amount to achieve antibody serum levels that exceed the amount required to saturate all of the CD4 of the primate being treated, with follow-up doses being given at times to maintain such excess over a period that induces tolerance in the primate against the antigen(s).

The CD4 antibody is a CD4 antibody that has a reduced effector (i.e. lytic) function as compared to human IgG1. The Ab has an Fc portion that is aglycosylated and has reduced binding to the Fc receptor.

The CD4 antibody with a reduced effector function is a non-depleting CD4 antibody. As used herein, "a non-depleting CD4 antibody" is a CD4 antibody that depletes less than 50% of CD4 cells and preferably less than 10% of CD4 cells.

In treating a primate and in particular a human, the CD4 antibody may be employed in combination with a pharmaceutically acceptable carrier. A composition that contains a CD4 antibody may include other ingredients, for example, stabilizers and/or other active agents.

The use of a CD4 antibody to induce tolerance against an antigen(s) in a primate in accordance with the present invention provides tolerance against one or more antigens and the primate is capable of immunologically responding to other antigens. Thus, in this respect, the primate is made tolerant to one or more antigens, and the immune system is capable of providing an immune response against other foreign antigens whereby the primate is not immunocompromised.

In the preferred embodiment where tolerance is induced against an antigen, the CD4 antibody is administered to the primate prior to, in conjunction with or subsequent to the antigen being delivered to the primate. In a preferred embodiment, the primate is provided with the CD4 antibody at a time such that the antibody is present in the primate. In a particularly preferred embodiment, the CD4 antibody is delivered to the primate prior to the primate coming into contact with the antigen to which the primate is to be tolerized or within a few hours or less than one day thereafter. In a preferred embodiment, the antibody is administered to the primate no more than about two, preferably no more than one day prior to the primate receiving the antigen.

As hereinabove indicated, in one embodiment, a primate is tolerized against a therapeutic protein that is to be used in treating the primate. Such therapeutic protein may be a therapeutic antibody (other than the CD4 antibody), which therapeutic antibody may be a human antibody, humanized antibody, chimeric antibody or a non-human antibody; an enzyme such as one used for replacement therapy; a hormone; clotting factor; a protein produced in gene therapy; a gene therapy delivery vehicle such as a vector used in gene therapy (for example, an adenovirus vector).

The foreign antigen(s) may be present in a transplanted organ, or in transplanted cells used in cell therapy, or in other tissue transplants, such as skin.

The treatment of a primate, in particular, a human, in order to tolerize the primate against a foreign antigen(s) by use of a CD4 antibody may be accomplished in some cases without adjunct therapy, such as a bone marrow transplant to promote acceptance of a foreign antigen and/or T-cell depletion and/or immunosuppression.

In some cases, adjunct therapy may also be employed. For example, as part of a transplant procedure, immunosuppression with an appropriate immunosuppressant may be used but by employing the present invention, chronic immunosuppression is not required. In addition, if used after or during the tolerizing procedure, in some cases, the immunosuppressant may be used with less than the amount required to provide for effective immunosuppression.

The Ab is a TRX1 antibody, as described in the claims, and such antibody is preferably used with the dosing regimen as hereinabove described.

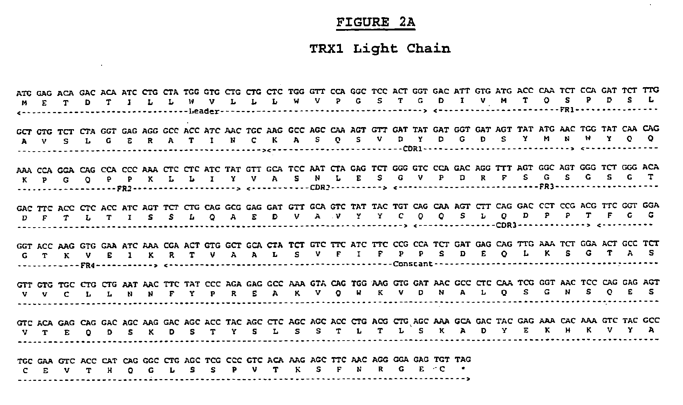

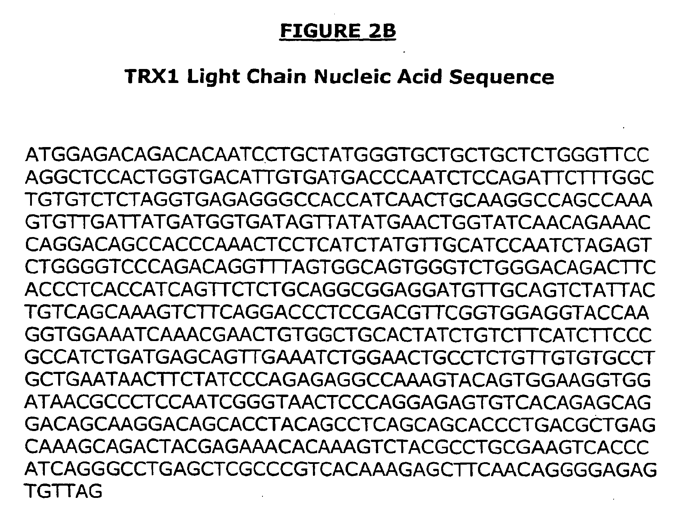

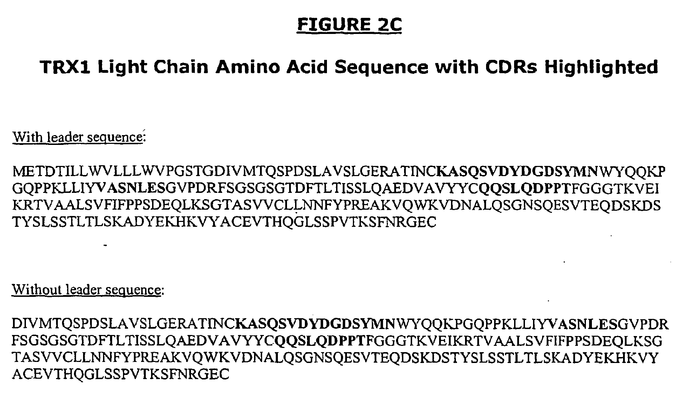

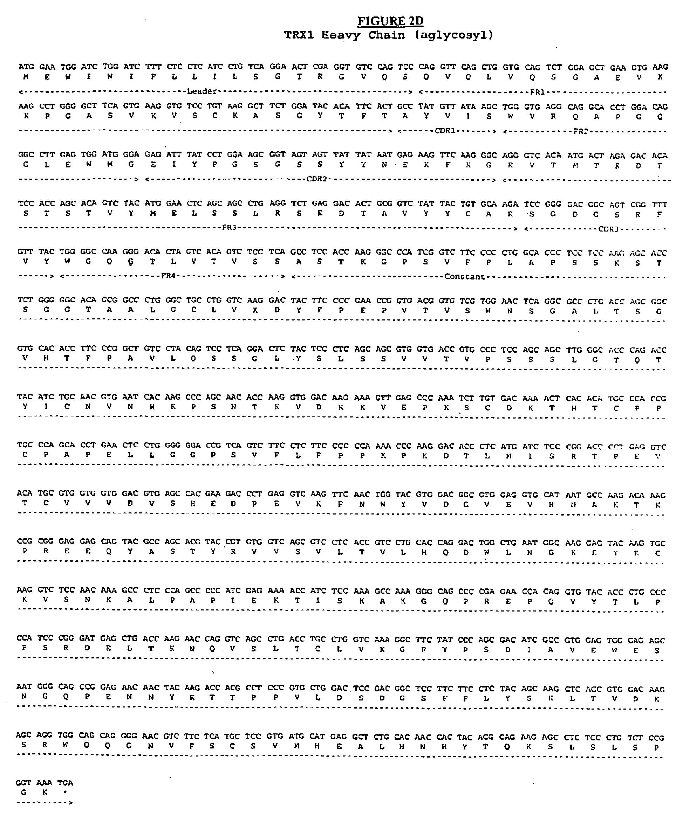

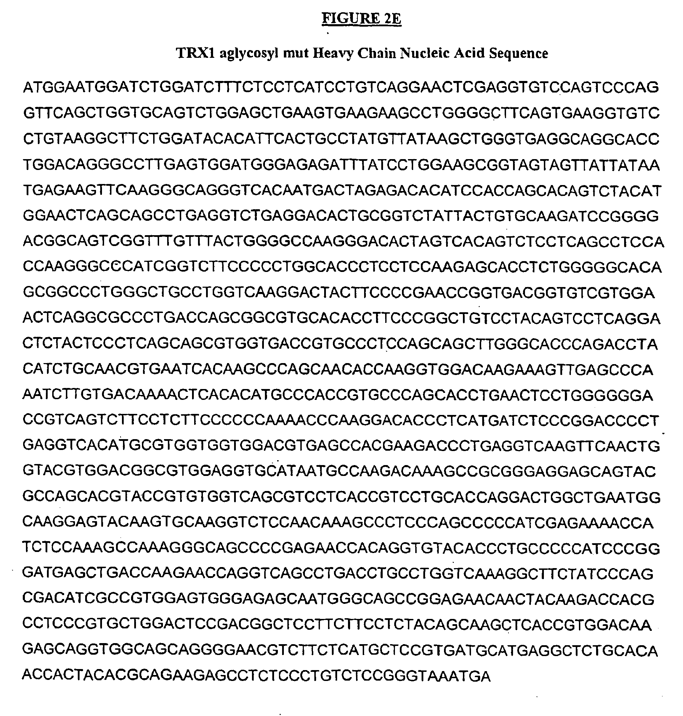

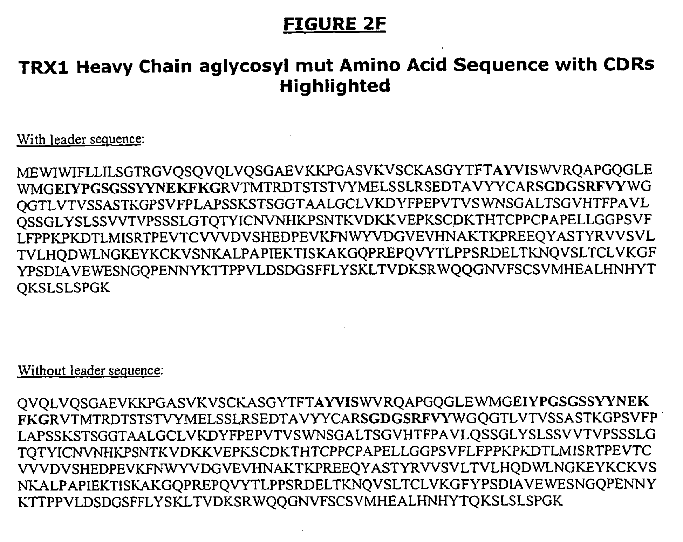

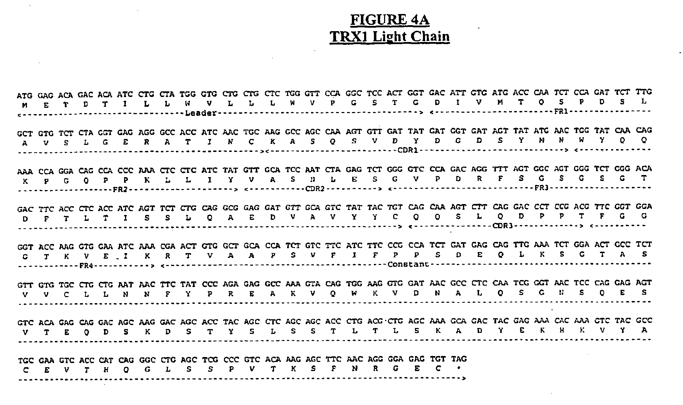

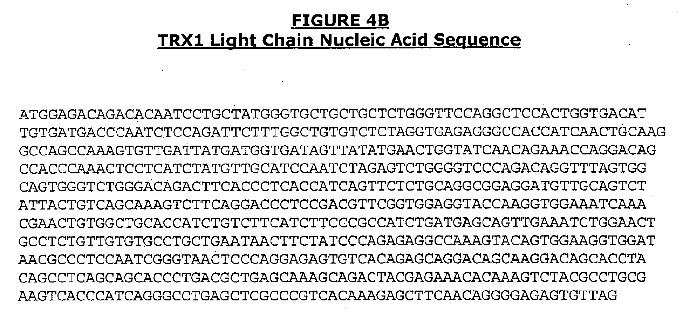

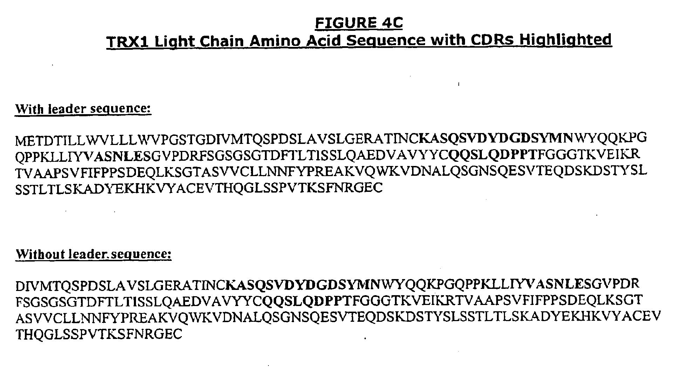

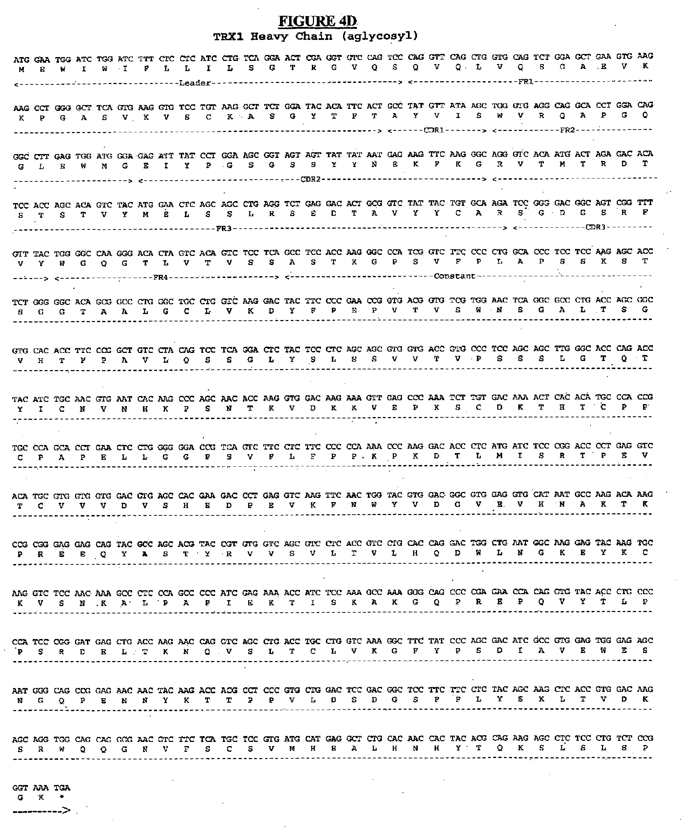

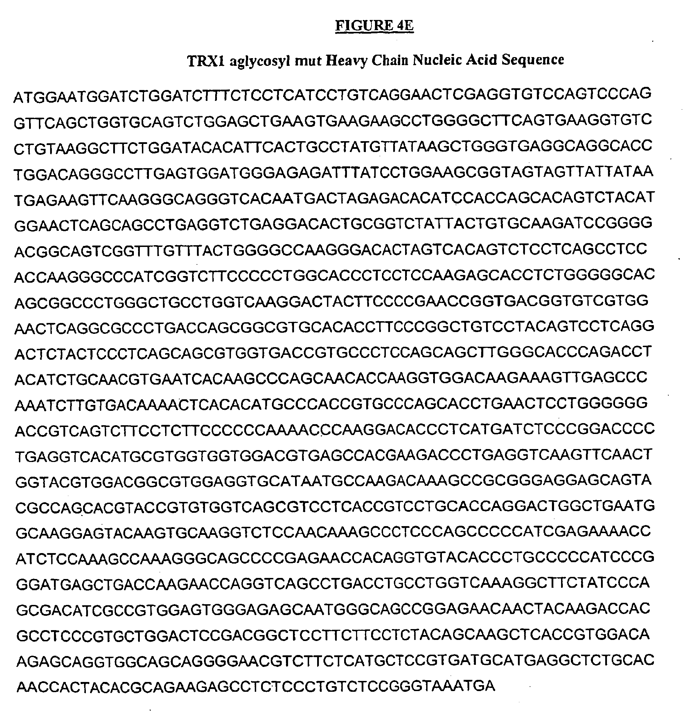

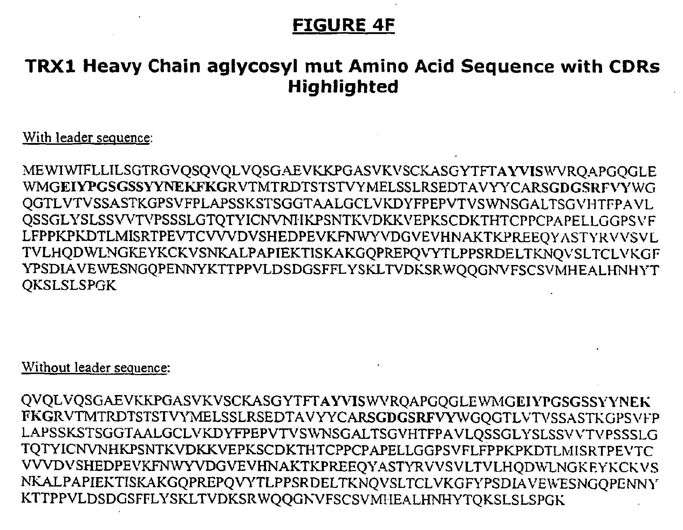

The antibody is hereinafter sometimes referred to as TRX1. The term "molecule" or "antibody" includes TRX1. The term "TRX1" includes the antibody shown in Figure 2 and the one of Figure 4, and those identical thereto which may be produced, for example, by recombinant technology.

As representative but non-limiting examples of such equivalent TRX1 antibodies there may be mentioned:
1) humanized antibodies that bind to the same epitope as TRX1;
2) humanized antibodies that have the same CDRs as TRX1 but which have a different humanized framework and/or a different human constant region;
3) humanized antibodies that bind to the same epitope as TRX1 in which one or more amino acids of one or more of the CDRs of TRX1 have been changed (preferably but not necessarily a conservative amino acid substitution) and in which the framework may be the same framework as TRX1 or have a different humanized framework or in which one or more of the amino acids of the framework region of TRX1 have been changed and/or in which the constant region may be the same as or different from TRX1;
4) humanized antibodies that bind to the same epitope as TRX1 wherein the antibody does not bind to the Fc region of the receptor.
5) humanized antibodies that bind to the same epitope as TRX1 wherein the CDRs thereof do not include a glycosylation site;
6) humanized antibodies that bind to the same epitope as TRX1 and that do not bind to the Fc region of the receptor and the CDRs do not include a glycosylation site;
7) a chimeric antibody that binds to the same epitope as TRX1; and
8) a murine antibody that binds to the same epitope as TRX1.

The antibodies that are equivalent to TRX1 may be used in the same manner and for the same purposes as TRX1.

The antibodies of the present invention may be used in a method treating an animal, in particular a human, especially for use in inhibiting, ameliorating, or reducing an immune response to an antigen, which may be a foreign antigen or a self antigen, including inducing tolerance to an antigen. The antibodies may be used to inhibit, ameliorate, or reduce an immune response to a Class I presented antigen and/or to a Class II resented antigen. The antibodies may be used to inhibit, ameliorate, or reduce an immune response to such antigens. In the case of a transplant, for example, Class I and Class II major histocompatibility (MHC) antigens and non-MHC or minor histocompatibility antigens may be presented. Apart from transplantation antigens, the antibodies may be used to inhibit, ameliorate, or reduce an immune response to globular proteins, glycoproteins such as immunoglobulins, materials carried on particles such as pollen proteins, polypeptides intended for therapeutic use such as interferon, Interleukin-2 or tumor necrosis factor, or hormone replacements such as lutenizing hormone, its analogues and antagonists. Further specific antigens to which an immune response can be inhibited, ameliorated, or reduced include synthetic peptide analogues of protein therapeutic agents which are used to aid in receptor blocking, and alloantigens. Alloantigens may be responsible for rejection of foreign tissue in tissue transplants or skin grafts. The term "antigen" as used herein is a compound or material that induces an immune response in an animal, in particular a human animal. The immune response may be a T-cell response which may or may not be accompanied by a humoral response.

The antibodies of the present invention inhibit and/or alter T-cell activation and proliferation and Applicant has found that such inhibition can be effected when adding the molecule or antibody either before or after an agent which stimulates T-cell activation.

The antibodies of the present invention have the characteristics of binding to an epitope of a CD4 antigen (CD4 positive human T-cells), but it is to be understood, however, that although the antibody is believed to function by binding to a CD4 antigen on T-cells, the antibody may function by binding to a CD4 antigen on other cells; e.g., monocytes. As a result, the ability of such molecules or antibodies to inhibit and/or alter T-cell activation or proliferation may or may not be effected through binding to CD4 positive cells, although Applicant presently believes that the mechanism of action involves binding of the molecule or antibody to CD4 positive cells.

In accordance with another aspect of the present invention, there is provided a method of preventing and/or inhibiting an on-going immune response in human patients through the administration to the patient of the antibody, hereafter referred to as TRX1.

Although Applicants do not want to limit the invention to any theoretical reasoning, it is believed that the mechanism which enables the monoclonal antibody of this invention to inhibit or prevent or reduce or ameliorate the severity of an immune response, and to inhibit and/or alter the activation and proliferation of effector T cells, is the fact that the TRX1 antibody either decreases the density of CD4 expressed on T-cell surfaces which are capable of participating in an immune reaction, and thus decreases the number of functional CD4 +effector T lymphocytes; and/or affects signal transduction and thus decreases the number of functional CD4 + effector T lymphocytes. It is believed that these mechanisms of action are responsible for not only the prevention of immune responses, but also the reduction in severity of on-going immune responses. In addition, the TRX1 antibody inhibits natural killer (NK) cell activity in vitro. This is pertinent to the present invention because it is believed that a non-MHC restricted cytotoxic mechanism such as NK cell activity has been implicated in graft versus host disease.

The term "inhibit" as used herein throughout this application is intended to mean prevention, or inhibition, or reduction in severity, or amelioration of an immune response to one or more antigens. The antigen may be a foreign antigen or a self antigen. The term "graft" as used herein for purposes of this application shall mean any and all transplantation, including but not limited to, allograft and xenograft transplantation. Such transplantation may by way of example include, but not be limited to, transplantation of cells, bone marrow, tissue, solid-organ, bone, etc.

The term "immune response(s)" as used herein is intended to mean immune responses dependent upon T cell activation and proliferation which includes both cellular effects and T cell dependent antibodies which may be elicited in response to, by way of example and not limitation: (i) grafts, (ii) graft versus host disease, and (iii) autoantigens resulting in autoimmune diseases, which by way of example include but are not limited to rheumatoid arthritis, systemic lupus, multiple sclerosis, diabetes mellitus, etc.

In a preferred embodiment, the antibody does not bind to Fc receptors through the Fc region of the antibody and the CDRs do not include a glycosylation site.

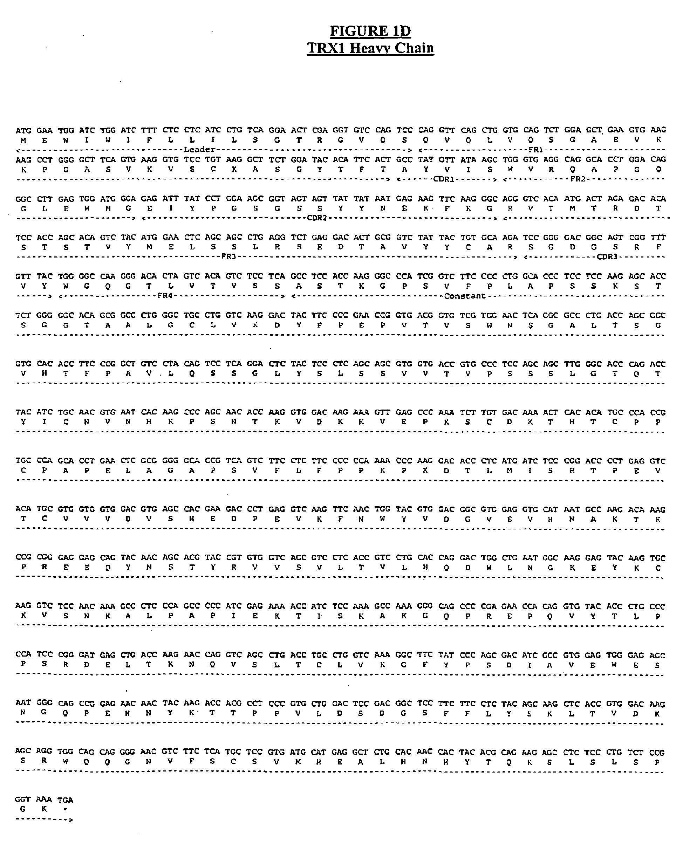

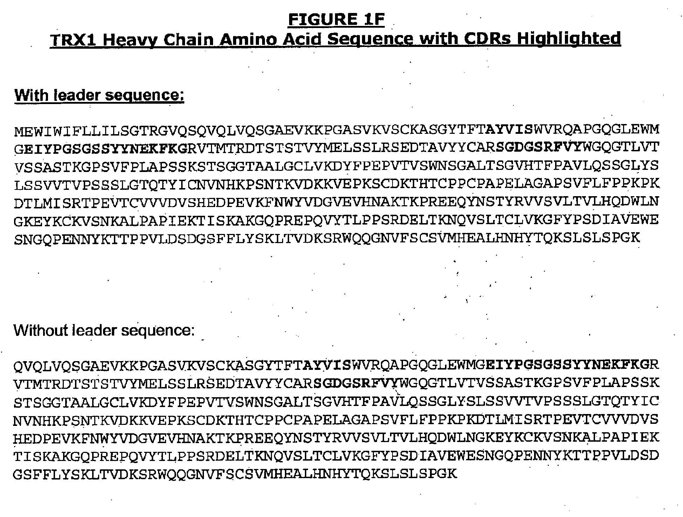

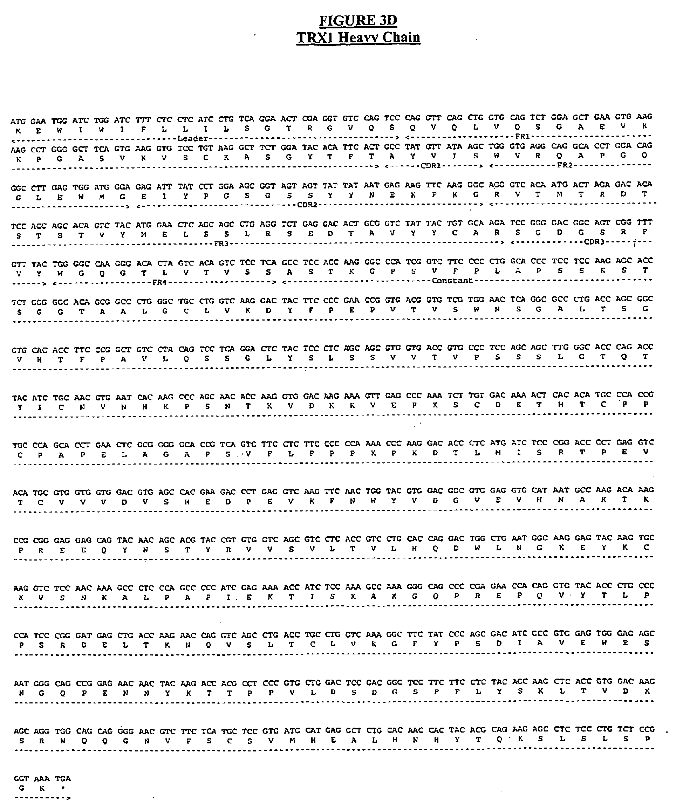

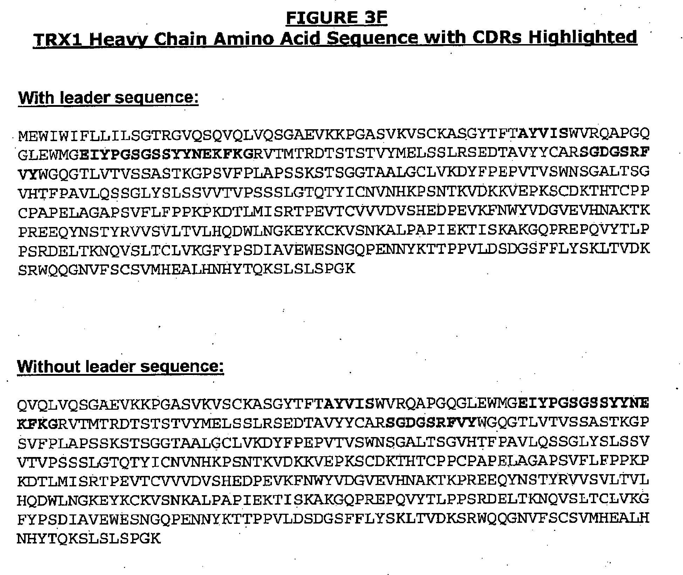

The heavy chain constant region does not include a glycosylation site. An example of a heavy chain sequence which includes a glycosylation site is shown in Figures 1D and 1F and Figures 3D and 3F. An example of a heavy chain sequence which does not include a glycosylation site is shown in Figures 2D and 2F and in Figures 4D and 4F.

The term "antibody" as used herein includes monoclonal antibodies as well as antibodies prepared by recombinant techniques, i.e. humanized antibodies.

Another embodiment of the present invention provides for a method of treating a patient who is to receive or has received a graft transplant with an effective amount of TRX1.

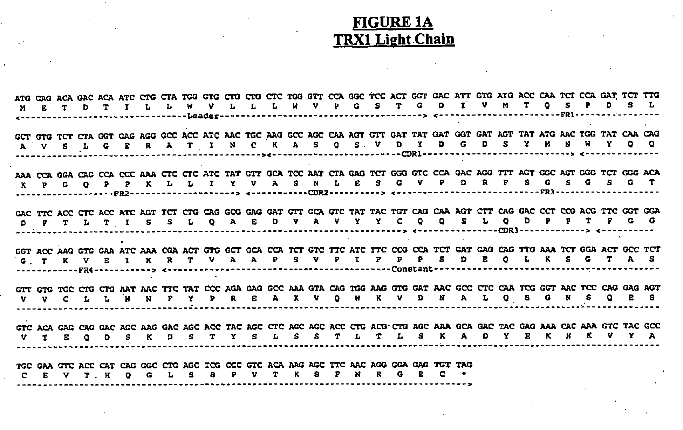

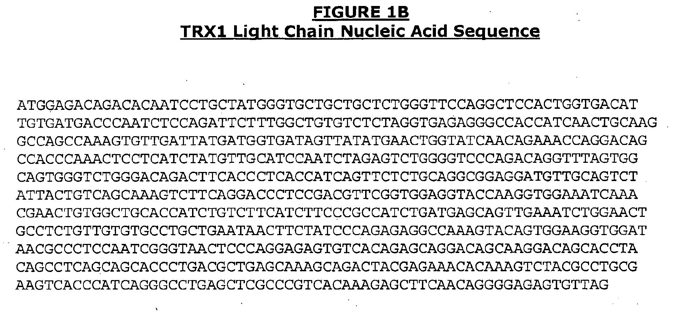

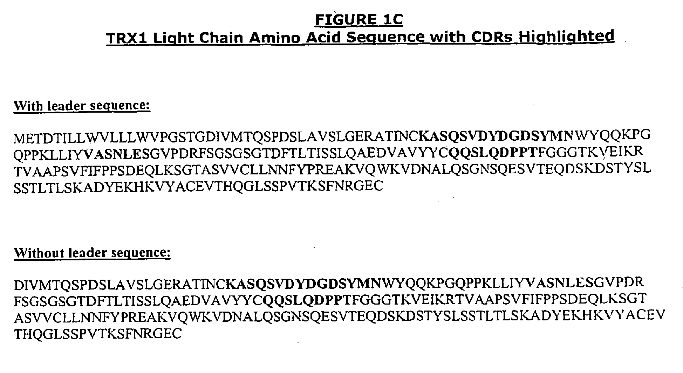

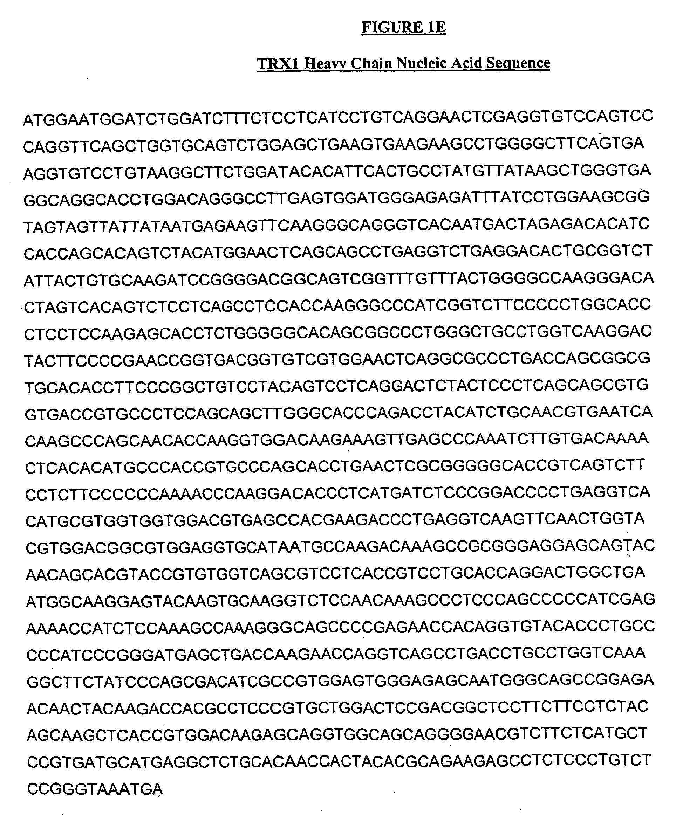

In one embodiment, the antibody is TRX1 which is a humanized antibody that includes modified constant regions of a human antibody, and light and heavy chain framework and CDR regions, in which the framework regions of the light and heavy chain variable regions correspond to the framework regions of the light and heavy chain variable region of a human antibody, and the CDRs derived from a mouse monoclonal antibody designated NSM4.7.2.4. An example of a TRX1 antibody is shown in Figure 1. Figure 1A shows the amino acid and DNA sequences for the TRX1 light chain. Figure 1B shows the TRX1 light chain nucleic acid sequence. Figure 1C shows the TRX1 light chain amino acid sequence with the CDRs highlighted. Figure 1D shows the amino acid and DNA sequences for the TRX1 heavy chain which includes a glycosylation site. Figure 1E shows the TRX1 heavy chain nucleotide sequence. Figure 1F shows the TRX1 heavy chain amino acid sequences, which include a glycosylation site, with the CDRs highlighted. It will be understood that TRX1 of Figure 1 is not part of the invention.

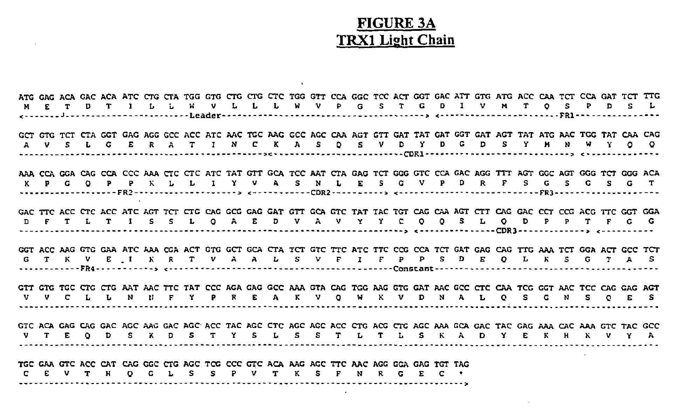

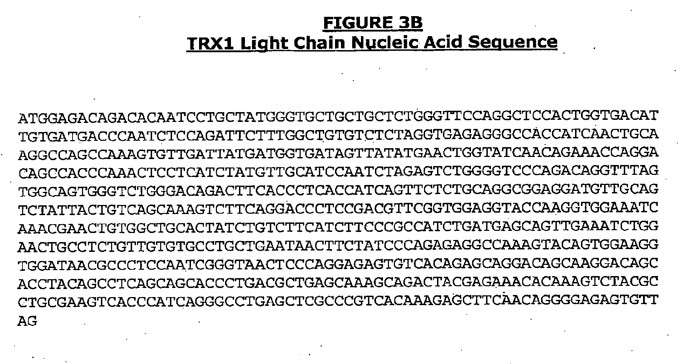

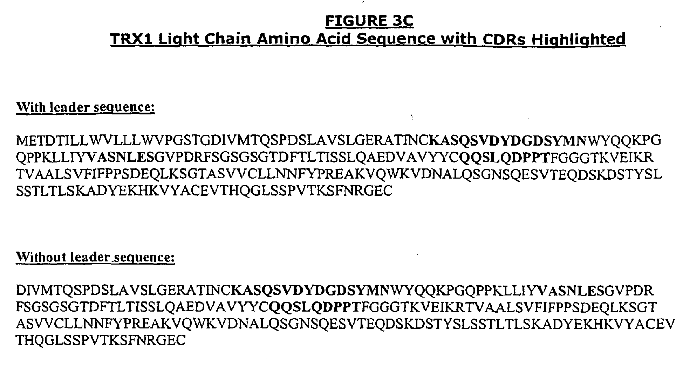

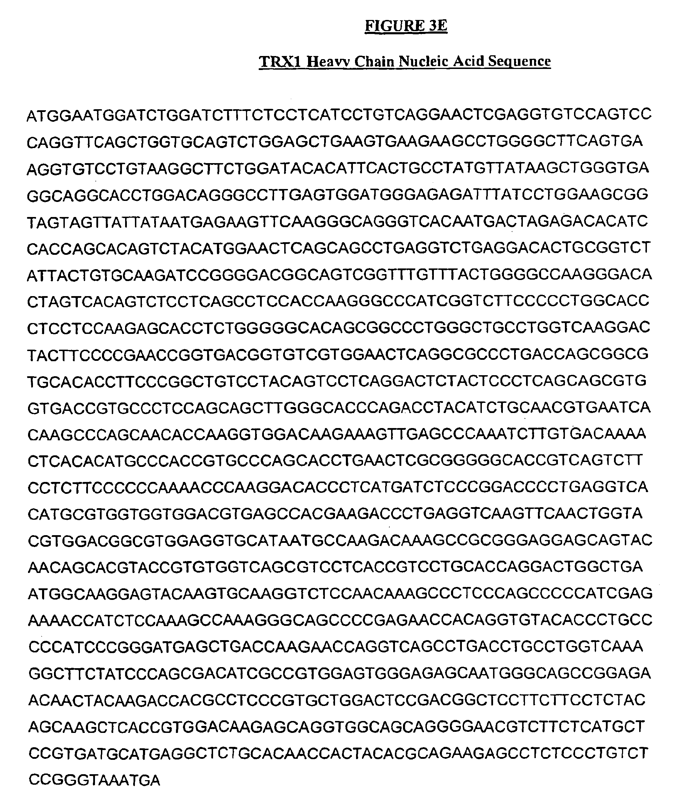

Another example of TRX1 is a humanized antibody that includes modified constant regions of a human antibody, and light and heavy chain framework and CDR regions, in which the framework regions of the light and heavy chain variable regions correspond to the framework regions of the light and heavy chain variable region of a human antibody, and the CDRs derived from a mouse monoclonal antibody designated NSM4.7.2.4. This example of the TRX1 antibody is shown in Figure 3. Figure 3A shows the amino acid and DNA sequences for the TRX1 light chain. Figure 3B shows the TRX1 light chain nucleic acid sequence. Figure 3C shows the TRX1 light chain amino acid sequence with the CDRs highlighted. Figure 3D shows the amino acid and DNA sequences for the TRX1 heavy chain which includes a glycosylation site. Figure 3E shows the TRX1 heavy chain nucleotide sequence. Figure 3F shows the TRX1 heavy chain amino acid sequences, which include a glycosylation site, with the CDRs highlighted. It will be understood that TRX1 of Figure 3 is not part of the invention.

One embodiment of the TRX1 antibody of the invention is shown in Figure 2. Figure 2A shows the amino acid and DNA sequences for the light chain. Figure 2B shows the light chain nucleic acid sequence. Figure 2C shows the light chain amino acid sequence with the CDRs highlighted. Figure 2D shows the amino acid and DNA sequences for the heavy chain. Figure 2E shows the heavy chain nucleotide sequence. Figure 2F shows the heavy chain amino acid sequences with the CDRs highlighted.

Another embodiment of the TRX1 antibody of the invention is shown in Figure 4. Figure 4A shows the DNA and amino acid sequences for the light chain. Figure 4B shows the light chain nucleic acid sequence. Figure 4C shows the light chain amino acid sequence with the CDRs highlighted. Figure 4D shows the amino acid and DNA sequences for the heavy chain. Figure 4E shows the heavy chain nucleotide sequence. Figure 4F shows the heavy chain amino acid sequences with the CDRs highlighted.

In the figures, amino acid residue 1 is the first amino acid, in each of the heavy and light chains, after the leader sequence. It also is the first residue in FR1 in the sequences.

The preparation of TRX1 humanized antibody suitable for the purposes of the present invention should be apparent to those skilled in the art from the teachings herein. Such antibody may be prepared by recombinant techniques known to those skilled in the art.

The antibodies of the present invention may be used to inhibit an immune response in an animal by administering the antibody in an amount effective to inhibit such immune response.

For example, in some cases, treatment with a therapeutic agent includes an immune response against the therapeutic agent. As representative examples of such therapeutic agents there may be mentioned monoclonal antibodies such as ReoPro and OKT3, enzymes for replacement therapy such as, but not limited to, glucocerebrosidase for Gaucher's disease and clotting factors such as Factor VIII, and products of gene therapy and gene therapy delivery vehicles such as adenovirus derived vectors.

In accordance with an aspect of the present invention, the antibody of the invention is administered to a patient that is to be treated with such therapeutic agent, with the antibody being administered in an amount effective to inhibit the immune response against the therapeutic agent. The antibody may be administered prior to, in combination with, or subsequent to administration of the therapeutic agent. The method of administration is dependent on a variety of factors, including, but not limited to, the specific indication, specific therapeutic agent and optimal dosing schedule If administered prior to the administration of the therapeutic agent, the antibody is administered from about 1 hour to about 10 days prior to the administration of the therapeutic agent, preferably from about 1 hour to about 24 hours prior to the administration of the therapeutic agent. If administered after the administration of the therapeutic agent, the antibody is administered from about 1 hour to about 10 days after the administration of the therapeutic agent, preferably from about 1 hour to about 24 hours after the administration of the therapeutic agent.

The amount of antibody administered, the dosing schedule and the number of times that the antibody is administered is dependent upon the therapeutic agent and the regimen used for treating a patient with the therapeutic agent. In general, the antibody may be used in an amount from 0.1 milligram to 3 grams per dose.

The antibody of the present invention may also be used to inhibit an immune response against a self-antigen and/or a foreign antibody, e.g., against a transplant (for example, transplant rejection) and/or to inhibit or ameliorate an immune response of a graft against a host.

The antibody of the present invention may also be used to inhibit an immune response against gene therapy products as well as an immune response against gene therapy delivery vehicles such as adenovirus derived vectors which limit the effectiveness of the gene therapy.

Thus, an immune response to an antigen in a host can be inhibited, ameliorated, or reduced by administering TRX1 antibody of the invention along with the antigen. A patient may be given a tissue transplant such as an organ transplant or a bone marrow transplant and may be given TRX1 antibody of the invention along with the transplant to inhibit rejection thereof. Also, tolerance may be induced to an antigen already possessed by a patient. Long-term specific tolerance can be induced to a self-antigen or antigens in order to treat autoimmune diseases.

Persistent or periodic antigen presence is required to maintain tolerance. A tissue graft, for example, supplies the antigen to maintain tolerance to itself. In the case of extraneous foreign antigens such as allergens, antigen "reminders" can be given at regular intervals.

The antibody may be administered in vivo in accordance with the present invention to inhibit the activation and proliferation of T-cells, and decrease the density of functional CD4 expression on the cell surface and/or affect signal transduction thereby reducing the functionality of CD4+ T lymphocytes and/or the number of CD4⁺ T lymphocytes.

Thus, for example, in an in vivo procedure, such antibodies are administered to prevent and/or inhibit an immune response and thereby inhibit T cell activation and proliferation.

The antibody may be administered ex vivo in accordance with the present invention to decrease the density of functional CD4⁺ expression on the cell surface and/or affect signal transduction, thus reducing the functionality of CD4+ T lymphocytes and/or the number of CD4⁺ cells of the donor cells. By way of example and not limitation, in an ex vivo procedure, such antibodies would be infused into donor bone marrow prior to transplantation to prevent the onset of graft versus host disease upon transplantation.

The antibody is generally administered in a pharmaceutically acceptable carrier. As representative examples of such carriers, there may be mentioned normal saline solution, buffers, etc. Such pharmaceutical carriers are well known in the art and the selection of a suitable carrier is deemed to be within the scope of those skilled in the art from the teachings contained herein.

The TRX1 antibody of the present invention may be administered in vivo intravenously, subcutaneously, or by intramuscular administration, etc.

As hereinabove indicated, TRX1 antibody of the present invention is administered in vivo in an amount effective to inhibit an immune response against an antigen(s). The term "an effective amount" for purposes of this Application shall mean that amount of antibody capable of producing the desired effect. In general, such antibody is administered in an amount of at least 0.1 milligram per dose. It is to be understood that lower amounts could be used. In addition after the initial treatment, the hereinabove described amounts may be reduced for subsequent treatments, if any.

The TRX1 antibody of the present invention may be employed to induce tolerance to an antigen. The term "tolerance", as used herein, means that a T-cell non-response persists against an antigen after stopping the antibody treatment, even in the case of challenge. If needed, however, booster or reinforcing doses of the antibody may be given in order to maintain such tolerance.

The techniques of the present invention for inhibiting the activation of T-cells may be employed alone or in combination with other techniques, drugs or compounds for inhibiting the activation of T-cells or inhibiting graft rejection or graft versus host disease or in treating various autoimmune diseases Examples may include drugs such as rapamycin and cyclosporin, or other immunomodulatory compounds including monoclonal antibodies directed against co-stimulatory molecules such as CD2, CD8 and CD28, as well as monoclonal antibodies directed against adhesion molecules.

The antibodies of the present invention also may be employed in a method of selecting for or determining the presence of CD4 positive cells in a sample, such a blood sample, for example. In such method, a sample is contacted with the antibody, and the presence of CD4 positive cells is determined, and/or CD4 positive cells then can be selected or isolated form the sample.

The antibody of the invention is employed to induce tolerance against an antigen(s) in a primate (in particular a human).

### EXAMPLES

The invention now will be described with respect to the following examples.

### EXAMPLE 1

A cDNA library was constructed from the mouse hybridoma NSM 4.7.2.4 using the Superscript plasmid system (Gibco/BRL, cat. no. 82485A) according to the manufacturer's suggested protocol. Heavy and light chain cDNAs were cloned from the library by DNA hybridization using as probes rat heavy and light chain gene cDNAs from the rat hybridoma YTS 177.

The rat heavy and light chain gene cDNAs of YTS 177 were isolated from the expression vector pHA Pr-1 as BamH1/Sal 1 fragments and labeled with ³²P and used independently to screen the NSM 4.7.2.4. cDNA library using standard molecular biology techniques (Sambrook, et al., Molecular Cloning, A. Laboratory Manual, 3rd edition, Cold Spring Harbor Laboratory Press, Cold Spring Harbor, New York (2001); Ausubel, et al., Current Protocols in Molecular Biology, John Wiley & Sons, New York (2001).) Sequence analysis of the cDNAs derived from the NSM 4.7.2.4 cDNA library confirmed the NSM 4.7.2.4 heavy chain to be mouse gamma-1 subclass and the NSM 4.7.2.4 light chain to be kappa. The NSM 4.7.2.4 heavy and light V regions (VH and VL, respectively) were reshaped to the human VH and VL regions with the "best fit" or highest sequence similarity in the framework regions to that of the mouse. For the light chain, human antibody HSIGKAW (from EMBL) with a sequence similarity of 79%, was used (LA Spatz et al., 1990 J. Immunol. 144:2821-8). The sequence of HSIGKAW VL is:

| | |
|---|---|
| **D** | start of framework 1 |
| **Q** | changed to G |

For the heavy chain, human antibody A32483 (From GenBank) with a sequence similarity of 74%, was used (Larrick, et al., Biochem. Biophys. Res. Comm., Vol. 160, pgs. 1250-1256 (1989)). The sequence of A32483 VH is:

| | |
|---|---|
| **Q** | start of framework 1 |

For the humanization process, anti-CD4 light chain clone 77.53.1.2 (insert size 1 kb) and anti-CD4 heavy chain clone 58.59.1 (insert size 1.7kb) were chosen from the cDNA library and inserts isolated from the pSport vector as Sal I/Not I fragments and cloned into M13mp18 vector to produce single stranded DNA for sequencing and template for mutagenesis. The humanization of NSM 4.7.2.4 was performed by site-directed mutagenesis of the mouse cDNA using a kit from Amersham International (RPN 1523) according to the manufacturer's suggested protocol.

Mutagenesis of the VL gene framework regions was performed using five oligonucleotides ranging in length from 29 to 76 bases. The oligos used were:
**Primer #1998 76 bases**
**Primer #1999 29 bases**
   5'-TGA ACT GGT ATC AAC AGA AAC CAG GAC AG
**Primer #2000 28 bases**
   5'-AGA GTC TGG GGT CCC AGA CAG GTT TAG T
**Primer #2001 42 bases**
**Primer #2008 52 bases**

The oligos were phosphorylated and mutagenesis performed in three steps using no more than two oligos per step to introduce changes according to the following procedure:
(1) Annealing phosphorylated mutant oligos to ssDNA template
(2) Polymerization
(3) Filtration to remove single-stranded DNA
(4) Nicking non mutant strand with Nci I
(5) Digestion of non-mutant strand with Exo III
(6) Repolymerization of gapped DNA
(7) Transformation of competent JM101
(8) Sequencing of clones

Mutations were confirmed by single strand DNA sequencing using M13 primers -20 and -40 and also the mutagenic primers # 1999 and # 2000.

A Sal I site at the 5' end of the variable region was changed to Hind III by linker oligos #2334 and #2335 to allow cloning of the variable region as a Hind III/Kpn I fragment into the light chain constant region of CAMPATH-1H.
**Primer #2334 24 bases**
5'-AGC TTT ACA GTT ACT GAG CAC ACA
**Primer #2335 24 bases**
5'-TCG ATG TGT GCT CAG TAA CTG TAA

Mutagenesis of the VH gene framework regions was performed using five oligonucleotides ranging in length from 24 to 75 bases. The oligos used were:
**Primer #2003 75 bases**
**Primer # 2004 52 bases**
**Primer #2005 60 bases**
**Primer #2006 44 bases**
**Primer #2007 24 bases** 5'-GCC AAG GGA CAC TAG TCA CTG TGT

Mutagenesis was carried out as described above for the light chain again using no more than two oligos at a time to introduce the changes. Mutations were confirmed by single strand DNA sequencing using M13 primers -20 and -40 as well as the mutagenic primers #2002 and #2004.

Primer #2002 was used to correct a reading frame error in starting clone 58.59.1.
**Primer #2002 39 bases**
5'-ACT CTA ACC ATG GAA TGG ATC TGG ATC TTT CTC CTC ATC

Primer #2380 was used to correct extra mutation added by #2004 which was missed in the first sequencing.
**Primer #2380 39 bases**
5'-TCA CTG CCT ATG TTA TAA GCT GGG TGA GGC AGG CAC CTG

As with the light chain, the heavy chain 5' Sal I site was changed to Hind III using linker oligo's #2334 and #2335 to allow cloning of the heavy chain variable region as Hind III/ Spe I (site introduced by primer #2007) fragment into the heavy chain constant region of CAMPATH-1H.

### Construction of heavy chain

The following samples of DNA were used

### 1. Plasmid 1990

Human gamma-1 heavy chain constant region gene cloned into pUC18 (obtained from Martin Sims, Wellcome Foundation Ltd).

### 2. Plasmid 2387

Reshaped heavy chain of NSM 4.7.2.4 containing human framework regions and mouse gamma 1 constant region.

A Sal I site in the reshaped CD4 heavy chain was altered to a Hind III site. The variable region gene was excised by digestion with Hind III/Spe I and ligated with the constant region gene in plasmid 1990 to give a complete humanized heavy chain (plasmid 2486). The heavy chain gene was cut out of this plasmid with Hind III/EcoR I and ligated with the expression vector pEE6.

### Construction of light chain

The following samples of DNA were used.

### 1. Plasmid 2028

CAMPATH-1H light chain gene cloned into M13mp18 at Sal I/BamH I restriction site.

### 2. Plasmid 2197

Reshaped light chain of NSM 4.7.2.4 containing human framework regions and mouse kappa constant region. A Kpn I site already had been introduced between variable and constant portions of this gene.

A Kpn I restriction site was introduced into the CAMPATH 1 H light chain gene corresponding to the site in plasmid 2197 and an EcoR I site was introduced at the 3' end of the constant region. The constant region gene was excised from this plasmid (2502) by digestion with Hind III/Kpn I.

Meanwhile a Sal I site in plasmid 2197 was changed to a Hind III site (this step had to be repeated because a frame-shift mutation inadvertently was introduced the first time). The new plasmid (2736) was digested with Hind III/Kpn I. The CD4 variable region fragment was cloned into a plasmid containing the kappa constant region gene from plasmid 2502 to give a complete humanized light chain (plasmid 2548). The light chain gene was cut out from this plasmid with Hind III/EcoR I and ligated with the expression vector pEE12 to give plasmid 2798.

### Ligation of heavy and light chains and expression in NSO cells

The heavy chain gene was excised from the pEE6 vector by digestion with Sal I/Bgl II and cloned into the light chain pEE12 vector which had been digested with BamH I/Sal I.

The final vector construct was checked by restriction digests with Hind III, EcoR I, Sal I, BamH I, Bgl II and Spe I for the presence of the expected fragments, including 700 bp light chain, 1400 bp heavy chain, 2300 bp fragment of pEE6 and 7000 bp fragment of pEE12.

The pEE12 vector was linearised by digestion with Sal I and transferred into NSO cells by electroporation, following a standard protocol (Celltech 1991) except that the selection medium was slightly modified, being based on IMDM rather than DMEM. Transfectants were selected in medium lacking glutamine, supplemented with dialysed FCS, ribonucleosides, glutamic acid, and asparagine as recommended.

The transfection mixes were cultured in three 96-well plates, and of 36 growing wells which were tested, 5 were strongly positive for production of human heavy and light chains (18 others were positive for one or other, or weakly positive for both).

A clone, designated SDG/B7B.A.7 was selected and stored frozen but no further characterization has been done on this wild type antibody.

### Construction of mutant IgG1 antibody designated to abolish effector functions

Due to concerns about side effects of other CD4 antibodies reported in various clinical trials, it was considered desirable to avoid the possibility of engaging Fc receptors. Human IgG4 is thought to have minimal Fc binding or complement-activating ability. However, experiments have show that it does engage Fc receptors in some individuals (Greenwood et al., Eur. J. Immunol., Vol. 23, pgs. 1098-1104, 1993), and clinical studies with a human IgG4 variant to CAMPATH-1H have demonstrated an ability to kill cells *in vivo* (Isaacs et al., Clin. Exp. Immunol., Vol. 106, pgs. 427-433 (1996)). To eliminate the possibility of binding Fc receptors, constructs were made with mutations in the IgG1 heavy chain constant region.

TRX 1 has the mutations Leu²³⁶ to Ala and Gly²³⁸ to Ala, as shown in Figures 1D and 1 E and Figures 3D and 3E. These particular residues were chosen because they were predicted to disrupt maximally binding to all three types of human Fc receptors for IgG. Either mutation is sufficient to reduce binding to Fc(RI (Woof, et al., Mol. Immunol, Vol. 332, pgs. 563-564, 1986; Duncan, et al., Nature, Vol. 332, pgs. 563-564 1988; Lund, et al., J. Immunol, Vol. 147, pgs. 2657-2662 1991) or Fc(RII (Lund et al., 1991; Sarmay et al., Mol. Immunol., Vol. 29, pgs. 633-639 1992) whereas Gly²³⁸ to Ala has the biggest effect on binding to Fc(RIII (Sarmay et al., 1992).

The following samples of DNA were used.

### 1. Plasmid 2555 and Plasmid 2555 Mut.

Humanized V_{H} region of NSM 4.7.2.4 cloned into pEE6 expression vector at a Hind III/Spe I restriction site. Plasmid 2555 then was mutated by site directed mutagenesis such that amino acid residue Asn¹⁰¹ was changed to Asp¹⁰¹, as shown in Figures 1D and 1 E and Figures 3D and 3E. The resulting plasmid is plasmid 2555 Mut.

### 2. Plasmid 2798

Humanized V_{H} region of NSM 4.7.2.4 joined to human kappa constant regions to give approx 700 bp fragment cloned into pEE12 expression vector at a Hind III/EcoR I.

### 3. Plasmid MF4260

Human IgG1 heavy chain associated with the humanized CD18 V_{H} region, having the mutations Leu²³⁶ to Ala and Gly²³⁸ to Ala as well as a Spe I restriction site introduced into framework region 4, cloned into pUC18.

The purpose of the Spe I restriction site is to allow separation and recombination of different variable regions.

The CD18 V_{H} region gene was excised from plasmid MF 4260 by digestion with Spe I and Hind III and the remaining vector, now having only the relevant heavy chain constant region, was purified using Geneclean. It was ligated with the humanized V_{H} region DNA of NSM 4.7.2.4 which had been isolated from plasmid 2555 Mut in the same way. The product was used to transform "Sure" cells and colonies were checked for the presence of the expected 1400 bp complete heavy chain insert.

The complete V_{H} and constant region insert was excised from the pUC vector by digestion with Hind III and EcoR I. The 1400 bp fragment was purified using Qiaexll (Qiagen) and then ligated in turn into the vector pEE6, which had previously been cut with the same enzymes.

The next step was to excise the CD4 heavy chain genes from the pEE6 vector and clone them into pEE12, already containing the humanized CD4 light chain gene (plasmid 2798). The pEE6 vector was digested with Sal I and Bgl II and the pEE12 vector was digested with Sal I and BamH I to create the appropriate sites for re-ligation.

The final vector construct was checked by restriction digests with Hind III, EcoR I, Sal I and Spe I for the presence of the expected fragment, *i*.*e*., 700 bp light chain, 1400 bp heavy chain, 2300 bp fragment of pEE6, and 7000 bp fragment of pEE12.

The pEE12 vector was linearized by digestion with Sal I and transfected into NSO cells by electroporation as above. The transfection mixes were cultured in six 96-well plates, and of 90 growing wells which were tested, all were positive for production of human heavy and light chains. At this stage a sample of the pEE12 vector DNA was digested with Sal I, precipitated with ethanol and transferred to the Therapeutic Antibody Centre (TAC).

### Target Cells For Final Transfection

NSO cells were obtained directly from the ECACC (clone CB1782, accession number 85110503). A master cell bank (MCB) was prepared at the Therapeutic Antibody Centre, Churchill Hospital, Oxford, England.

### Transfection and Selection Of Final Transfectant

The pEE12 vector was transfected into NSO cells from the MCB by electroporation as hereinabove described. A total of 2x10⁷ cells were transfected with 80 µg of linearized plasmid DNA in a final volume of 2.0ml. The transfection mix was plated out in twelve 96-well plates and fed with selective medium according to the standard protocol (The Cell Tech Glutamine Synthetase Gene Expression System, Version 2 - Expression from Myeloma Cells, Revision 6.) Six plates received selective medium containing 10(M methionine sulfoximine (MSX).

### Purification of the antibody

Culture supernatant is purified by using a Biopilot chromatography system (Pharmacia) in three steps as follows:
(1) Affinity chromatography on a column of Protein A-Sepharose Fast Flow
(2) Ion exchange chromatography on S-Sepharose Fast Flow
(3) Size exclusion chromatography on Superdex 20.

The purified product was filtered and pooled into a single biocontainer.

Throughout the purification process, precautions are taken to ensure that the system remains aseptic. All buffers and reagents are passed through a 0.2 micron membrane filter and the purified product is also passed through a 0.2 micron filter before being pooled. After a batch of antibody has been processed, the entire chromatography system and columns are sanitized with 0.5M NaOH, washed with sterile PBS and stored in 20% ethanol. Before it is used again, the ethanol is washed out with sterile PBS and a complete trial run is carried out. Samples of buffers and column eluates are checked for endotoxin level.

### Example 2

### Construction of TRX1 Antibody Starting from Nucleotide Sequence

### Cloning of Human Constant Regions

### Heavy Chain Constant Region

The human gamma 1 heavy chain constant region (IgG1) is amplified from human leukocyte cDNA (QUICK-Clone^{™} cDNA Cat. No. 7182-1, Clontech) using the following primer set and cloned into pCR-Script (Stratagene). The plasmid containing the human gamma 1 heavy chain constant region in pCR-Script is designated pHCγ-1.

| | |
|---|---|
| ***primer hc***γ***-1*** | |
| 5' primer: | |
| ***primer hc***γ***-2*** | |
| 3' primer: | |

Non-Fc binding mutations (Leu²³⁶Ala, Gly³⁸Ala ) are made in the heavy chain constant region by site-directed mutagenesis using the following primer and the Transformer^{™} Site-Directed Mutagenesis Kit from Clontech (Cat. No. K1600-1). The plasmid containing the human gamma 1 heavy chain non-Fc binding mutant constant region in pCR-Script is designated pHCγ-1Fcmut.

| | |
|---|---|
| ***primer hc***γ***-3*** | |
| | Fc mut oligo: |

### Light Chain Constant Region

The human kappa light chain constant region is amplified from human leukocyte cDNA (QUICK-Clone^{™} cDNA Cat. No. 7182-1, Clontech) using the following primer set and cloned into pCR-Script (Stratagene). The plasmid containing the human kappa light chain constant region in pCR-Script is designated pLCκ-1.

| | |
|---|---|
| ***primer Ic***κ***-1*** | |
| 5' primer: | |
| ***primer Ic***κ***-2*** | |
| 3' primer: | |

### Synthesis, Contruction and Cloning of TRX1 Variable Regions

The heavy and light chain variable regions are constructed from a set of partially overlapping and complementary synthetic oligonucleotides encompassing the entire variable regions. The oligonucleotide set used for each variable region is shown below.

### Heavy Chain Variable Region Synthetic Oligonucleotides

### Coding Strand Heavy Chain Variable Region Primers

***primer hv-1* (1 - 72) + 6 nucleotide linker**
***primer hv-2* (120 - 193)**
***primer hv-3* (223 - 292)**
***primer hv-4* (322 - 399)**

### Non-Coding Strand Heavy Chain Variable Region Primers

***primer hv-5* (140 - 51)**
***primer hv-6* (246 - 170)**
***primer hv-7* (342 - 272)**

### Light Chain Variable Region Synthetic Oligonucleotides

### Coding Strand Light Chain Variable Region Primers

***primer lv-1* (1 - 63) + 6 nucleotide linker**
***primer lv-2* (93 - 158)**
***primer lv-3* (184 - 248)**
***primer lv-4* (275 - 340)**

### Non-Coding Strand Light Chain Variable Region Primers

***primer lv-5* (109-43)**
***primer lv-6* (203-138)**
***primer lv-7* (294-228)**
***primer lv-8* (378-319)**

After HPLC purification and removal of organic solvents the oligonucleotides are resuspended in TE pH8.0 and phosphorylated. An aliquot of each oligonucleotide in the respective variable region set then are combined in equal molar amounts. The oligonucleotide mixtures are heated to 68°C for 10 minutes and allowed to cool slowly to room temperature. The annealed oligonuceotides then are extended to produce double stranded variable region DNA fragments. For the extension, dNTPs are added to a final concentration of 0.25 mM followed by an appropriate volume of 5X T4 DNA polymerase buffer [165 mM Tris acetate, pH 7.9, 330 mM sodium acetate, 50mM magnesium acetate, 500 (g/ml BSA, 2.5mM DTT] and 4 units of T4 DNA polymerase. The mixture is incubated at 37°C for 1 hour followed by heat inactivation of the T4 DNA polymerase at 65°C for 5 minutes.

The double stranded DNA is ethanol precipitated and resuspended in the same volume of TE pH 8.0. An appropriate volume of 5X T4 DNA ligase buffer [250mM Tris-HCl, pH7.6, 50mM MgCl₂, 5mM ATP, 5mM DTT, 25% w/v polyethylene glycol-8000] then is added to the double stranded DNA followed by 2 units of T4 DNA ligase and the mixture incubated for 1 hour at 37°C to ligate the extended fragments. The T4 DNA ligase then is heat inactivated at 65°C for 10 minutes. The variable region DNA fragments then are phenol extracted, ethanol precipitated, and resuspended in TE, pH 8.0 and cloned into pCR-Script (Stratagene). The resulting plasmid containing the heavy chain variable region is designated pHV-1 and the plasmid containing the light chain variable region was designated pLV-1.

The final heavy and light chain expression vectors are constructed in pcDNA 3.1 (Invitrogen). For the heavy chain expression vector, the Fc mutated constant region is released from plasmid pHC-1Fcmut by digestion with Spe I and EcoR I and isolated by agarose gel electrophoresis. The heavy chain variable region is released from plasmid pHV-1 by digestion with Hind III and Spe I and isolated by agarose gel electrophoresis. The two fragments in equal molar amounts are ligated into the Hind III/EcoR I sites of pcDNA3.1(+) (Invitrogen) using standard molecular biology techniques. The resulting TRX1 heavy chain expression vector is designated pTRX1/HC.

Similarly, for the light chain expression vector, the light chain constant region is released from plasmid pLC-1 by digestion with Kpn I and Hind III followed by agarose gel purification. The light chain variable region is released from pLV-1 by digestion with EcoR I and Kpn I followed by agarose gel purification. The two light chain fragments in equal molar amounts are ligated into the EcoR I/Hind III sites of pcDNA3.1 (-) (Invitrogen) using standard molecular biology techniques yielding the TRX1 light chain expression vector pTRX1/LC.

For production of TRX1 antibody, the TRX1 heavy chain and TRX1 light chain expression plasmids are cotransfected into CHO cells using standard molecular biology techniques.

### Example 3

A humanized antibody is shown in Figures 2A, 2C, 2D, and 2F is produced by a procedure similar to that of Example 1. The humanized antibody is an aglycosylated antibody.

### Example 4

A humanized antibody as shown in Figures 4A, 4C, 4D and 4F is produced by a procedure similar to that of Example 1. The humanized antibody is an aglycosylated antibody.

### Example 5

A mixed lymphocyte reaction (MLR) is used to generate human lymphocytes primed to recognize foreign human histocompatibility antigens. To generate this reaction human peripheral blood lymphocytes are isolated from heparinized whole blood from two different individuals (Donor A and Donor B) using Ficoll density gradient centrifugation or a similar method. Lymphocytes from Donor B are adjusted to 10⁷/ml in RPMI 1640 media with no serum but containing 50ug/ml of mitomycin C. The cells are incubated at 37°C for 30 minutes and are then washed out of the media with mitomycin C in three centrifugations in RPMI 1640 with 10% Donor A plasma. Cells from Donor A, which have not been treated with mitomycin C, are adjusted to 4 x 10⁶/ml in RPMI 1640 with 10% Donor A plasma. After washing, the mitomycin C treated lymphocytes from Donor B are adjusted to 4 x 10⁶/ml in RPMI with 10% Donor A plasma. Equal volumes of Donor A and Donor B cells are combined and placed into the compound to be tested ("Test Compound") suitably sized tissue culture flasks. Flasks with and without Test Compound are then incubated at 37°C in 5% CO₂ in air for 7-10 days. This is the primary mixed lymphocyte reaction.

It can be observed that the cells in the primary MLR will become activated and begin to divide between days 3-7 and following a period of active proliferation, the cells will return to a more resting state. The time of this can vary, however, cells will usually return to rest between days 7-10. Once cells appear to be at rest, cells from primary MLR flasks with and without Test Compound can be recovered by centrifugation and resuspended in RPMI 1640 with 10% Donor A plasma at 4 x 10⁶/ml. Fresh PBL can be prepared by Ficoll density centrifugation from heparinized whole blood from Donor B and again inactivated with mitomycin C, the inactivated Donor B cells are adjusted to 4 x 10⁶/ml in RPMI 1640 with 10% Donor A plasma. For the second MLR, equal volumes of primary MLR cells (cells from the primary MLR which was performed in the absence of Test Compound) are mixed with mitomycin C inactivated Donor B cells.

If the primary MLR cells (cells obtained from an MLR which was performed in the absence of Test Compound) are labeled with CFSE, a green fluorescent dye which is imported into living cells where it is acted upon by an enzyme and then reacts with cellular proteins, the number of cell divisions experienced over time by the labeled cells is reflected in the reduction of green label associated with each cell. If CFSE labeled MLR cells are stimulated in a secondary MLR to which has been added cells derived from the Test Compound treated primary MLR at a ratio of 2:1 to 10:1 MLR to Test Compound derived MLR cells, inhibition of the proliferation of the CFSE labeled MLR cells will be observed in the secondary MLR within 3-4 days of stimulation when compared with proliferation of CFSE labeled MLR cells stimulated in the secondary MLR in the absence of the Test Compound derived cells.

The cells produced in the primary MLR and secondary MLR (as well as cells provided in control MLRs) are analyzed for CD4+ CD25+ cells as hereinabove described.

When TRX1 was tested as hereinabove described, as compared to control, CD4+ CD25+ cells were reduced by more than 60% in the primary MLR and by more than 20% in the secondary MLR, and in the secondary MLR, as compared to control, the production of IL-2, IL-4 and IL-12 was essentially eliminated and the production of IL-5,IL-13, IFN, gamma and TNF alpha was reduced by more than 50%.

### Example 6

The induction of antigen specific immunological tolerance in non-human primates by the use of the non-depleting anti-CD4 monoclonal antibody, TRX1, was demonstrated in the following study. Baboons (*Papio anubus*) were randomly divided into seven groups of three animals. The seven groups were comprised of four experimental groups designated Groups 4, 6, 7 and 8 and three control groups designated Groups 1, 5, and 9. The study was comprised of 2 phases, an Immunization/Tolerization phase followed by a Challenge phase.

For the Immunization/Tolerization phase of the study, Groups 4, 6, 7 and 8 were immunized with 3 doses of Antigen 1 (10 mg/kg in saline), one dose each on day 0, day 4, and day 8. Antigen 1 is polyvalent aggregated horse IgG (Antivenin) given intravenously (i.v.) for the first dose and subcutaneously (SC) for all subsequent doses. During this phase of the protocol: groups 4, 6, 7 & 8 also received 4 doses i.v. of the non-depleting anti-CD4 monoclonal antibody, TRX1, as follows: Group 4 received 4 doses of 20 mg/kg at day -1, day 4, day 8 and day 12; Group 6 received 1 mg/kg of TRX 1 on day -1, day 3, day 8, and day 12. Group 7 received 10 mg/kg on day -1, day 3, day 8 and day 12. Group 8 received 40 mg/kg on day -1, day 3, day 8, and day 12.

Control Groups 1 and 5 were treated as follows during the Immunization/Tolerization phase of the protocol: Group 1 received 3 doses of Antigen 1 at 10 mg/kg, one dose each on day 0, day 4, and day 8. Group 5 received 4 doses i.v. of TRX1 antibody, 20 mg/kg, one dose each on day -1, day 4, day 8, and day 12. Group 9 received four doses IV of TRX 1 antibody, 40 mg/kg, one dose each on day -1, day 4, day 8, and day 12.

Blood was collected prior to each injection of Antigen 1 and/or TRX1 and weekly thereafter to assess serum level of TRX1 by ELISA, the pharmacodynamic effects of TRX1 treatment on the level of circulating lymphocyte subsets, as well as the percentage of CD4 receptor occupancy by flow cytometry, and the baboon antibody response to Antigen 1 by ELISA.

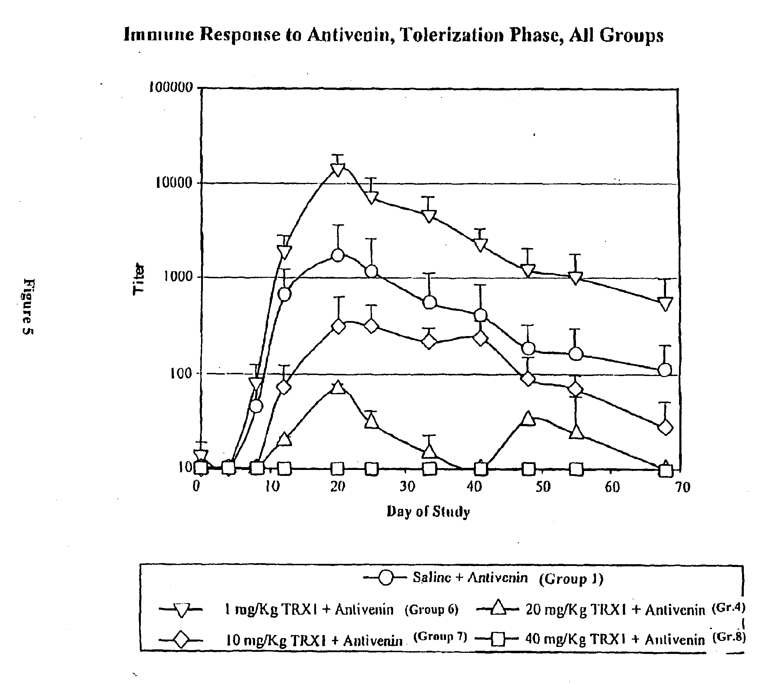

The immune response to antivenin for the first 68 days of the study for Groups 1, 4, 6, 7, and 8, as measured in antibody titer, is shown in Figure 5.

The Challenge phase of the study was initiated once the serum levels of TRX 1 reached undetectable levels. For the Challenge phase, all animals in all groups were challenged (Day 68) with Antigen 1 (10 mg/kg, SC) and Antigen 2 (1.7 ml/kg). Antigen 2 is a 10% saline solution of sheep red blood cells given IV for one dose. Challenges with Antigen 1 were repeated on day 95 and day 135 for Control Groups 5 and 9 and Test Groups 4 and 8. Blood was collected prior to each challenge to assess for serum levels of Antigen 1, TRX1, and the baboon antibody response to Antigen 1 and Antigen 2.

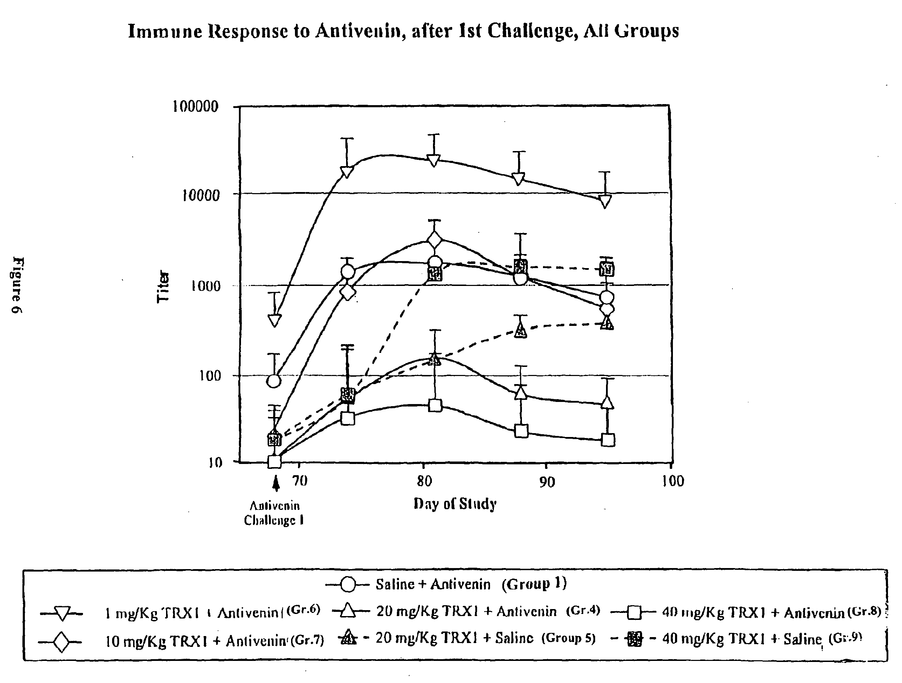

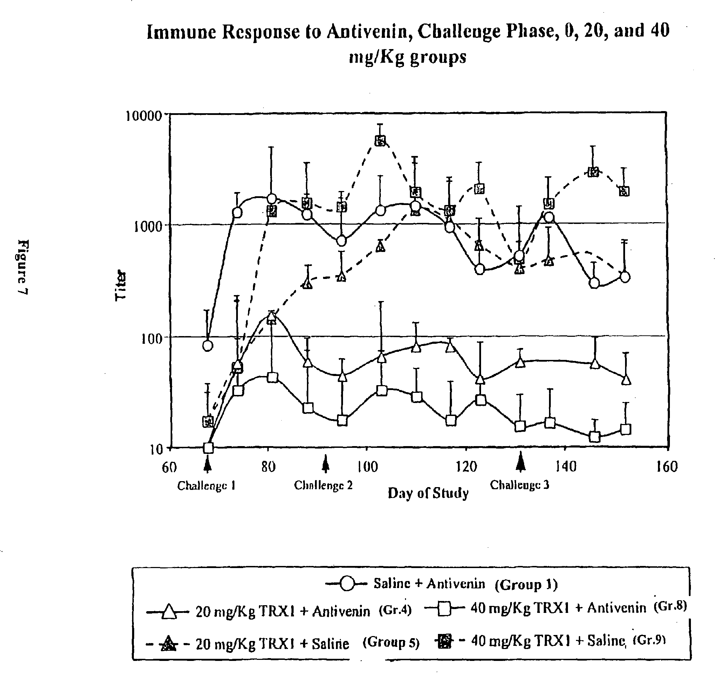

Figure 6 shows the immune response to antivenin, as measured in antibody titer, for all groups after the first challenge (days 68-95). Figure 7 shows the immune response to antivenin, as measured in antibody titer, for Groups 1, 4, 5, 8, and 9, after Challenge 1, Challenge 2, and Challenge 3.

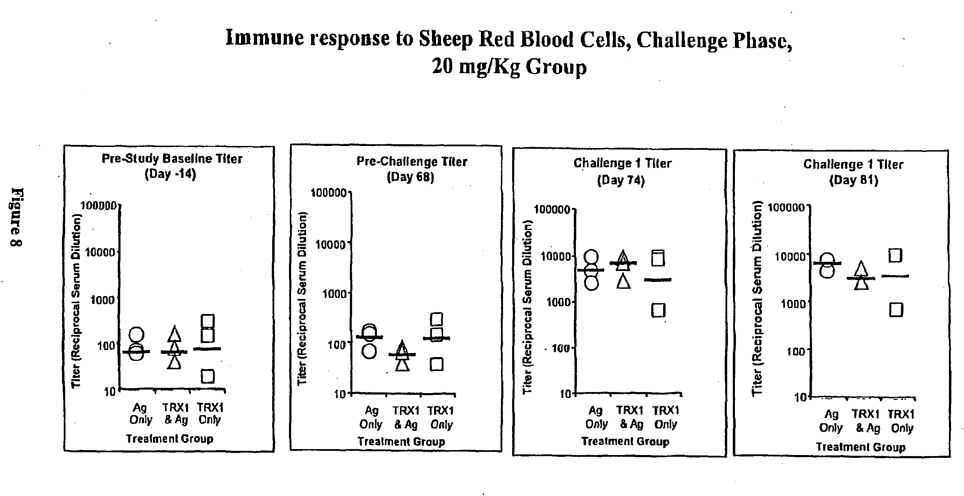

The results of the immune response of groups 1, 4 and 5 to sheep red-blood cells is shown in Figure 8.

## Claims

1. A humanized anti-CD4 non-depleting antibody comprising a Light Chain and a Heavy Chain, the Light Chain having the three Light Chain CDRs KASQSVDYDGDSYMN, VASNLES and QQSLQDPPT, and the Heavy Chain having the three Heavy Chain CDRs AYVIS, EIYPGSGSSYYNEKFKG and SGDGSRFVY, the antibody having an aglycosylated Fc portion.

2. An antibody according to claim 1, which has a human gamma 1 Heavy Chain constant region.

3. An antibody according to claim 1 or claim 2, which is human IgG1.

4. An antibody according to any preceding claim, further comprising the Light Chain and Heavy Chain framework regions of Figures 2 or 4.

5. An antibody according to claim 4, further comprising the Heavy Chain constant region of Figures 2 or 4.

6. An antibody according to claim 5, wherein said antibody is a humanized antibody identical to the humanized antibody shown in Figure 2.

7. An antibody according to claim 5, wherein said antibody is a humanized antibody identical to the humanized antibody shown in Figure 4.

8. An antibody according to any preceding claim, wherein said antibody includes CDRs that are free of a glycosylation site.

9. Use of an antibody according to any preceding claim in the manufacture of a medicament for the induction of tolerance to at least one antigen in a primate, wherein said antibody reduces the amount of CD4+ CD25+ cells produced in an in vitro primary mixed lymphocyte reaction and generates in said primary mixed lymphocyte reaction a cell population that reduces at least one of (x) the amount of CD4+ CD25+cells produced in vitro in at least one of a primary and secondary mixed lymphocyte reaction, and (y) the amount of at least one of IL-2, IL-4 and IL-12 in a secondary mixed lymphocyte reaction.

10. Use according to claim 9, wherein the compound reduces the amount of CD4⁺ CD25⁺ cells in a primary mixed lymphocyte reaction by at least 40%.

11. Use according to Claim 10, wherein the compound reduces the amount of CD4⁺ CD25⁺ cells in a primary mixed lymphocyte reaction by at least 60%.

12. Use according to Claim 10, wherein said cell population produced in the primary mixed lymphocyte reaction reduces the amount of CD4⁺ CD25⁺ cells produced in a secondary mixed lymphocyte reaction.

13. Use according to Claim 12, wherein the CD4⁺ CD25⁺ cells produced in the secondary mixed lymphocyte reaction are reduced by at least 20%.

14. Use according to Claim 13, wherein the CD4⁺ CD25⁺ cells produced in the secondary mixed lymphocyte reaction are reduced by at least 35%.

15. Use according to Claim 13, wherein in the secondary mixed lymphocyte reaction the generation of at least one of IL-2, IL-4 and IL-12 is reduced by at least 40%.

16. Use of an antibody according to any of claims 1-8 in the manufacture of a medicament for the induction of tolerance to at least one antigen in a primate, by administering the antibody, or fragment thereof, to the primate in an amount and for a time effective to induce tolerance against at least one antigen, said antibody or fragment being present in said primate when said antigen is present in said primate and being administered in an initial dose of at least 40 mg, said treating inducing tolerance against said at least one antigen, the antibody being an antibody that in a primary mixed lymphocyte reaction in vitro reduces the amount of CD4+ CD25+ cells produced in said mixed lymphocyte reaction.

17. Use according to Claim 16, wherein the initial dose is at least 70 mg.

18. Use according to Claim 17, wherein the initial dose is at least 400 mg.

19. Use according to Claim 18, wherein the initial dose is at least 500 mg.

20. Use according to Claim 16, wherein said antibody is administered in at least one follow-up dose and said follow-up dose is at least 40 mg.

21. Use according to Claim 16, wherein said at least one antigen is a foreign antigen.

22. A composition, comprising:
(a) an antibody according to any one of Claims 1-8; and
(b) an acceptable pharmaceutical carrier.

23. Use of an antibody according to any one of Claims 1-8 in the manufacture of a medicament for inducing tolerance to an antigen in a patient.

24. Use of an antibody according to any one of Claims 1-8 in the manufacture of a medicament for inhibiting an immune response in a patient.

25. Use of an antibody according to any one of Claims 1-8 in the manufacture of a medicament for inhibiting the rejection of a graft in a human patient.

26. Use according to Claim 25, wherein said graft is an organ.

27. An antibody according to any of claims 1-8 for use in a method of induction of tolerance as defined in any of claims 9-21 and 23, or inhibition as defined in any of claims 24-26.

## Patentansprüche

1. Humanisierter nicht-depletierender anti-CD4-Antikörper, umfassend eine leichte Kette und eine schwere Kette, wobei die leichte Kette die drei CDRs der leichten Kette KASQSVDYDGDSYMN, VASNLES und QQSLQDPPT aufweist und die schwere Kette die drei CDRs der schweren Kette AYVIS, EIYPGSGSSYYNEKFKG und SGDGSRFVY aufweist, wobei der Antikörper einen aglycosylierten Fc-Anteil aufweist.

2. Antikörper gemäß Anspruch 1, welcher eine humane konstante gamma-1-Region der schweren Kette aufweist.

3. Antikörper gemäß Anspruch 1 oder Anspruch 2, welcher humanes IgG1 ist.

4. Antikörper gemäß irgendeinem vorhergehenden Anspruch, weiterhin umfassend die Gerüstregionen der leichten Kette und schweren Kette der Figuren 2 oder 4.

5. Antikörper gemäß Anspruch 4, weiterhin umfassend die konstante Region der schweren Kette der Figuren 2 oder 4.

6. Antikörper gemäß Anspruch 5, wobei der Antikörper ein humanisierter Antikörper, identisch mit dem humanisierten Antikörper, gezeigt in Figur 2, ist.

7. Antikörper gemäß Anspruch 5, wobei der Antikörper ein humanisierter Antikörper, identisch mit dem humanisierten Antikörper, gezeigt in Figur 4, ist.

8. Antikörper gemäß irgendeinem vorhergehenden Anspruch, wobei der Antikörper CDRs einschließt, die frei von einer Glycosylierungsstelle sind.

9. Verwendung eines Antikörpers gemäß irgendeinem vorhergehenden Anspruch in der Herstellung eines Medikaments für die Induktion von Toleranz gegenüber mindestens einem Antigen bei einem Primaten, wobei der Antikörper die Menge von CD4⁺CD25⁺-Zellen, erzeugt in vitro in einer primären gemischten Lymphozytenreaktion, verringert und in der primären gemischten Lymphozytenreaktion eine Zellpopulation erzeugt, die mindestens eine von (x) der Menge von CD4⁺CD25⁺-Zellen, erzeugt in vitro in mindestens einer von einer primären und sekundären gemischten Lymphozytenreaktion, und (y) der Menge von mindestens einem von IL-2, IL-4 und IL-12 in einer sekundären gemischten Lymphozytenreaktion verringert.

10. Verwendung gemäß Anspruch 9, wobei die Verbindung die Menge von CD4⁺CD25⁺-Zellen in einer primären gemischten Lymphozytenreaktion um mindestens 40% verringert.

11. Verwendung gemäß Anspruch 10, wobei die Verbindung die Menge von CD4⁺CD25⁺-Zellen in einer primären gemischten Lymphozytenreaktion um mindestens 60% verringert.

12. Verwendung gemäß Anspruch 10, wobei die Zellpopulation, erzeugt in der primären gemischten Lymphozytenreaktion, die Menge von CD4⁺CD25⁺-Zellen, erzeugt in einer sekundären gemischten Lymphozytenreaktion, verringert.

13. Verwendung gemäß Anspruch 12, wobei die CD4⁺CD25⁺-Zellen, erzeugt in der sekundären gemischten Lymphozytenreaktion, um mindestens 20% verringert werden.

14. Verwendung gemäß Anspruch 13, wobei die CD4⁺CD25⁺-Zellen, erzeugt in der sekundären gemischten Lymphozytenreaktion, um mindestens 35% verringert werden.

15. Verwendung gemäß Anspruch 13, wobei in der sekundären gemischten Lymphozytenreaktion die Erzeugung von mindestens einem von IL-2, IL-4 und IL-12 um mindestens 40% verringert wird.

16. Verwendung eines Antikörpers gemäß irgendeinem der Ansprüche 1 bis 8 in der Herstellung eines Medikaments für die Induktion von Toleranz gegenüber mindestens einem Antigen bei einem Primaten durch Verabreichen des Antikörpers oder eines Fragments davon an den Primaten in einer Menge und für eine Zeit, wirksam, um Toleranz gegen mindestens ein Antigen zu induzieren, wobei der Antikörper oder das Fragment in dem Primaten vorhanden sind, wenn das Antigen in dem Primaten vorhanden ist, und in einer anfänglichen Dosis von mindestens 40 mg verabreicht werden, wobei das Behandeln Toleranz gegen das mindestens eine Antigen induziert, wobei der Antikörper ein Antikörper ist, der in einer primären gemischten Lymphozytenreaktion in vitro die Menge von CD4⁺CD25⁺-Zellen, erzeugt in der gemischten Lymphozytenreaktion, verringert.

17. Verwendung gemäß Anspruch 16, wobei die anfängliche Dosis mindestens 70 mg beträgt.

18. Verwendung gemäß Anspruch 17, wobei die anfängliche Dosis mindestens 400 mg beträgt.

19. Verwendung gemäß Anspruch 18, wobei die anfängliche Dosis mindestens 500 mg beträgt.

20. Verwendung gemäß Anspruch 16, wobei der Antikörper in mindestens einer Nachfolgedosis verabreicht wird und die Nachfolgedosis mindestens 40 mg beträgt.

21. Verwendung gemäß Anspruch 16, wobei das mindestens eine Antigen ein fremdes Antigen ist.

22. Zusammensetzung, umfassend:
(a) einen Antiköper gemäß irgendeinem der Ansprüche 1 bis 8; und
(b) einen verträglichen pharmazeutischen Träger.

23. Verwendung eines Antiköpers gemäß irgendeinem der Ansprüche 1 bis 8 in der Herstellung eines Medikaments zum Induzieren von Toleranz gegenüber einem Antigen bei einem Patienten.

24. Verwendung eines Antiköpers gemäß irgendeinem der Ansprüche 1 bis 8 in der Herstellung eines Medikaments zum Hemmen einer Immunantwort bei einem Patienten.

25. Verwendung eines Antiköpers gemäß irgendeinem der Ansprüche 1 bis 8 in der Herstellung eines Medikaments zum Hemmen der Abstoßung eines Transplantats bei einem menschlichen Patienten.

26. Verwendung gemäß Anspruch 25, wobei das Transplantat ein Organ ist.

27. Antiköper gemäß irgendeinem der Ansprüche 1 bis 8 zur Verwendung in einem Verfahren zur Induktion von Toleranz, wie definiert in irgendeinem der Ansprüche 9 bis 21 und 23, oder Hemmung, wie definiert in irgendeinem der Ansprüche 24 bis 26.

## Revendications

1. Anticorps anti-CD4 ne provoquant pas de déplétion humanisé comprenant une chaîne légère et une chaîne lourde, la chaîne légère présentant les trois CDRs de chaîne légère KASQSVDYDGDSYMN, VASNLES et QQSLQDPPT et la chaîne lourde présentant les trois CDRs de chaîne lourde AYVIS, EIYPGSGSSYYNEKFKG et SGDGSRFVY, l'anticorps possédant une portion Fc aglycosylée.

2. Anticorps selon la revendication 1, qui possède une région constante de chaîne lourde gamma 1 humaine.

3. Anticorps selon la revendication 1 ou la revendication 2, qui est une IgG1 humaine.

4. Anticorps selon l'une quelconque des revendications précédentes, comprenant en outre les régions d'encadrement de chaîne légère et de chaîne lourde de la Figure 2 ou 4.

5. Anticorps selon la revendication 4, comprenant en outre la région constante de chaîne lourde de la Figure 2 ou 4.

6. Anticorps selon la revendication 5, où ledit anticorps est un anticorps humanisé identique à l'anticorps humanisé montré dans la Figure 2.

7. Anticorps selon la revendication 5, où ledit anticorps est un anticorps humanisé identique à l'anticorps humanisé montré dans la Figure 4.

8. Anticorps selon l'une quelconque des revendications précédentes, où ledit anticorps inclut des CDRs qui sont exemptes d'un site de glycosylation.

9. Utilisation d'un anticorps selon l'une quelconque des revendications précédentes dans la fabrication d'un médicament pour l'induction d'une tolérance vis-à-vis d'au moins un antigène chez un primate, dans laquelle ledit anticorps réduit la quantité de cellules CD4⁺ CD25⁺ produites dans une réaction lymphocytaire mixte primaire in vitro et génère dans ladite réaction lymphocytaire mixte primaire une population cellulaire qui réduit au moins un de (x) la quantité de cellules CD4⁺ CD25⁺ produites in vitro dans au moins une d'une réaction lymphocytaire mixte primaire et d'une réaction lymphocytaire mixte secondaire, et (y) la quantité d'au moins un de IL-2, IL-4 et IL-12 dans une réaction lymphocytaire mixte secondaire.

10. Utilisation selon la revendication 9, dans laquelle le composé réduit la quantité de cellules CD4⁺ CD25⁺ dans une réaction lymphocytaire mixte primaire d'au moins 40%.

11. Utilisation selon la revendication 10, dans laquelle le composé réduit la quantité de cellules CD4⁺ CD25⁺ dans une réaction lymphocytaire mixte primaire d'au moins 60%.

12. Utilisation selon la revendication 10, dans laquelle ladite population cellulaire produite dans la réaction lymphocytaire mixte primaire réduit la quantité de cellules CD4⁺ CD25⁺ produites dans une réaction lymphocytaire mixte secondaire.

13. Utilisation selon la revendication 12, dans laquelle les cellules CD4⁺ CD25⁺ produites dans la réaction lymphocytaire mixte secondaire sont réduites d'au moins 20%.

14. Utilisation selon la revendication 13, dans laquelle les cellules CD4⁺ CD25⁺ produites dans la réaction lymphocytaire mixte secondaire sont réduites d'au moins 35%.

15. Utilisation selon la revendication 13, dans laquelle, dans la réaction lymphocytaire mixte secondaire, la génération d'au moins un de IL-2, IL-4 et IL-12 est réduite d'au moins 40%.

16. Utilisation d'un anticorps selon l'une quelconque des revendications 1 à 8 dans la fabrication d'un médicament pour l'induction d'une tolérance vis-à-vis d'au moins un antigène chez un primate, par l'administration de l'anticorps, d'un fragment de celui-ci, au primate dans une quantité et pendant un temps efficace pour induire une tolérance vis-à-vis d'au moins un antigène, ledit anticorps ou fragment étant présent dans ledit primate lorsque ledit antigène est présent dans ledit primate et étant administré dans une dose initiale d'au moins 40 mg, ledit traitement induisant une tolérance vis-à-vis dudit au moins un antigène, l'anticorps étant un anticorps qui dans une réaction lymphocytaire mixte primaire in vitro réduit la quantité de cellules CD4⁺ CD25⁺ produites dans ladite réaction lymphocytaire mixte.

17. Utilisation selon la revendication 16, dans laquelle la dose initiale est d'au moins 70 mg.

18. Utilisation selon la revendication 17, dans laquelle la dose initiale est d'au moins 400 mg.

19. Utilisation selon la revendication 18, dans laquelle la dose initiale est d'au moins 500 mg.

20. Utilisation selon la revendication 16, dans laquelle ledit anticorps est administré dans au moins une dose de rappel et ladite dose de rappel est d'au moins 40 mg.

21. Utilisation selon la revendication 16, dans laquelle ledit au moins un antigène est un antigène étranger.

22. Composition, comprenant:
(a) un anticorps selon l'une quelconque des revendications 1 à 8; et
(b) un support pharmaceutique acceptable.

23. Utilisation d'un anticorps selon l'une quelconque des revendications 1 à 8 dans la fabrication d'un médicament pour l'induction d'une tolérance vis-à-vis d'un antigène chez un patient.

24. Utilisation d'un anticorps selon l'une quelconque des revendications 1 à 8 dans la fabrication d'un médicament pour l'inhibition d'une réponse immunitaire chez un patient.

25. Utilisation d'un anticorps selon l'une quelconque des revendications 1 à 8 dans la fabrication d'un médicament pour l'inhibition du rejet d'une greffe chez un patient humain.

26. Utilisation selon la revendication 25, dans laquelle ladite greffe est un organe.

27. Anticorps selon l'une quelconque des revendications 1 à 8 pour une utilisation dans un procédé d'induction d'une tolérance comme il est défini dans l'une quelconque des revendications 9 à 21 et 23 ou d'inhibition comme il est défini dans l'une quelconque des revendications 24 à 26.


### SEQUENCE LISTING

<110> Cambrige University Technical Services Limited
   Isis Innovation Limited
   TolerRx, Inc.
<120> TRX1 Antibody and Uses Therefor
<130> WPP84098
<150> UK 0114517.6
   <151> 2001-06-14
<150> US 60/345,194
   <151> 2001-10-19
<150> US 60/373,470
   <151> 2002-04-18
<150> US 60/373,471
   <151> 2002-04-18
<150> UK 0122724.8
   <151> 2001-09-20
<160> 76
<170> PatentIn version 3.1
<210> 1
   <211> 717
   <212> DNA
   <213> Homo sapiens
<400> 1
   atggagacag acacaatcct gctatgggtg ctgctgctct gggttccagg ctccactggt      60
   gacattgtga tgacccaatc tccagattct ttggctgtgt ctctaggtga gagggccacc     120
   atcaactgca aggccagcca aagtgttgat tatgatggtg atagttatat gaactggtat     180
   caacagaaac caggacagcc acccaaactc ctcatctatg ttgcatccaa tctagagtct     240
   ggggtcccag acaggtttag tggcagtggg tctgggacag acttcaccct caccatcagt     300
   tctctgcagg cggaggatgt tgcagtctat tactgtcagc aaagtcttca ggaccctccg     360
   acgttcggtg gaggtaccaa ggtggaaatc aaacgaactg tggctgcacc atctgtcttc     420
   atcttcccgc catctgatga gcagttgaaa tctggaactg cctctgttgt gtgcctgctg     480
   aataacttct atcccagaga ggccaaagta cagtggaagg tggataacgc cctccaatcg     540
   ggtaactccc aggagagtgt cacagagcag gacagcaagg acagcaccta cagcctcagc     600
   agcaccctga cgctgagcaa agcagactac gagaaacaca aagtctacgc ctgcgaagtc     660
   acccatcagg gcctgagctc gcccgtcaca aagagcttca acaggggaga gtgttag        717
<210> 2
   <211> 238
   <212> PRT
   <213> Homo sapiens
<400> 2
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65                  70                  75                  80
   Gly Val Pro Asp Arg Phe Ser Gly Ser Gly Ser Gly Thr Asp Phe Thr
                   85                  90                  95
   Leu Thr Ile Ser Ser Leu Gln Ala Glu Asp Val Ala Val Tyr Tyr Cys
               100                 105                 110
   Gln Gln Ser Leu Gln Asp Pro Pro Thr Phe Gly Gly Gly Thr Lys Val
           115                 120                 125
   Glu Ile Lys Arg Thr Val Ala Ala Pro Ser Val Phe Ile Phe Pro Pro
       130                 135                 140
   Ser Asp Glu Gln Leu Lys Ser Gly Thr Ala Ser Val Val Cys Leu Leu
   145                 150                 155                 160
   Asn Asn Phe Tyr Pro Arg Glu Ala Lys Val Gln Trp Lys Val Asp Asn
                   165                 170                 175
   Ala Leu Gln Ser Gly Asn Ser Gln Glu Ser Val Thr Glu Gln Asp Ser
               180                 185                 190
   Lys Asp Ser Thr Tyr Ser Leu Ser Ser Thr Leu Thr Leu Ser Lys Ala
           195                 200                 205
   Asp Tyr Glu Lys His Lys Val Tyr Ala Cys Glu Val Thr His Gln Gly
       210                 215                 220
   Leu Ser Ser Pro Val Thr Lys Ser Phe Asn Arg Gly Glu Cys
   225                 230                 235
<210> 3
   <211> 716
   <212> DNA
   <213> Homo sapiens
<400> 3
<210> 4
   <211> 238
   <212> PRT
   <213> Homo sapiens
<400> 4
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65                  70                  75                  80
   Gly Val Pro Asp Arg Phe Ser Gly Ser Gly Ser Gly Thr Asp Phe Thr
                   85                  90                  95
   Leu Thr Ile Ser Ser Leu Gln Ala Glu Asp Val Ala Val Tyr Tyr Cys
               100                 105                 110
   Gln Gln Ser Leu Gln Asp Pro Pro Thr Phe Gly Gly Gly Thr Lys Val
           115                 120                 125
   Glu Ile Lys Arg Thr Val Ala Ala Pro Ser Val Phe Ile Phe Pro Pro
       130                 135                 140
   Ser Asp Glu Gln Leu Lys Ser Gly Thr Ala Ser Val Val Cys Leu Leu
   145                 150                 155                 160
   Asn Asn Phe Tyr Pro Arg Glu Ala Lys Val Gln Trp Lys Val Asp Asn
                   165                 170                 175
   Ala Leu Gln Ser Gly Asn Ser Gln Glu Ser Val Thr Glu Gln Asp Ser
               180                 185                 190
   Lys Asp Ser Thr Tyr Ser Leu Ser Ser Thr Leu Thr Leu Ser Lys Ala
           195                 200                 205
   Asp Tyr Glu Lys His Lys Val Tyr Ala Cys Glu Val Thr His Gln Gly
       210                 215                 220
   Leu Ser Ser Pro Val Thr Lys Ser Phe Asn Arg Gly Glu Cys
   225                 230                 235
<310> 5
   <211> 218
   <212> PRT
   <213> Homo sapiens
<400> 5
<210> 6
   <211> 1404
   <212> DNA
   <213> Homo sapiens
<400> 6
   atggaatgga tctggatctt tctcctcatc ctgtcaggaa ctcgaggtgt ccagtcccag    60
   gttcagctgg tgcagtctgg agctgaagtg aagaagcctg gggcttcagt gaaggtgtcc   120
   tgtaaggctt ctggatacac attcactgcc tatgttataa gctgggtgag gcaggcacct   180
   ggacagggcc ttgagtggat gggagagatt tatcctggaa gcggtagtag ttattataat   240
   gagaagttca agggcagggt cacaatgact agagacacat ccaccagcac agtctacatg   300
   gaactcagca gcctgaggtc tgaggacact gcggtctatt actgtgcaag atccggggac   360
   ggcagtcggt ttgtttactg gggccaaggg acactagtca cagtctcctc agcctccacc   420
   aagggcccat cggtcttccc cctggcaccc tcctccaaga gcacctctgg gggcacagcg   480
   gccctgggct gcctggtcaa ggactacttc cccgaaccgg tgacggtgtc gtggaactca   540
   ggcgccctga ccagcggcgt gcacaccttc ccggctgtcc tacagtcctc aggactctac   600
   tccctcagca gcgtggtgac cgtgccctcc agcagcttgg gcacccagac ctacatctgc   660
   aacgtgaatc acaagcccag caacaccaag gtggacaaga aagttgagcc caaatcttgt   720
   gacaaaactc acacatgccc accgtgccca gcacctgaac tcgcgggggc accgtcagtc   780
   ttcctcttcc ccccaaaacc caaggacacc ctcatgatct cccggacccc tgaggtcaca   840
   tgcgtggtgg tggacgtgag ccacgaagac cctgaggtca agttcaactg gtacgtggac   900
   ggcgtggagg tgcataatgc caagacaaag ccgcgggagg agcagtacaa cagcacgtac   960
   cgtgtggtca gcgtcctcac cgtcctgcac caggactggc tgaatggcaa ggagtacaag  1020
   tgcaaggtct ccaacaaagc cctcccagcc cccatcgaga aaaccatctc caaagccaaa  1080
   gggcagcccc gagaaccaca ggtgtacacc ctgcccccat cccgggatga gctgaccaag  1140
   aaccaggtca gcctgacctg cctggtcaaa ggcttctatc ccagcgacat cgccgtggag  1200
   tgggagagca atgggcagcc ggagaacaac tacaagacca cgcctcccgt gctggactcc  1260
   gacggctcct tcttcctcta cagcaagctc accgtggaca agagcaggtg gcagcagggg  1320
   aacgtcttct catgctccgt gatgcatgag gctctgcaca accactacac gcagaagagc  1380
   ctctccctgt ctccgggtaa atga                                          1404
<210> 7
   <211> 467
   <212> PRT
   <213> Homo sapiens
<400> 7
   Met Glu Trp Ile Trp Ile Phe Leu Leu Ile Leu Ser Gly Thr Arg Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Ala Tyr Val Ile Ser Trp Val Arg Gln Ala Pro Gly Gln Gly Leu
       50                  55                  60
   Glu Trp Met Gly Glu Ile Tyr Pro Gly Ser Gly Ser Ser Tyr Tyr Asn
   65                  70                  75                  80
   Glu Lys Phe Lys Gly Arg Val Thr Met Thr Arg Asp Thr Ser Thr Ser
                   85                  90                  95
   Thr Val Tyr Met Glu Leu Ser Ser Leu Arg Ser Glu Asp Thr Ala Val
               100                 105                 110
   Tyr Tyr Cys Ala Arg Ser Gly Asp Gly Ser Arg Phe Val Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Ser Leu Gly Thr Gln Thr Tyr Ile Cys Asn Val Asn His
       210                 215                 220
   Lys Pro Ser Asn Thr Lys Val Asp Lys Lys Val Glu Pro Lys Ser Cys
   225                 230                 235                 240
   Asp Lys Thr His Thr Cys Pro Pro Cys Pro Ala Pro Glu Leu Ala Gly
                   245                 250                 255
   Ala Pro Ser Val Phe Leu Phe Pro Pro Lys Pro Lys Asp Thr Leu Met
               260                 265                 270
   Ile Ser Arg Thr Pro Glu Val Thr Cys Val Val Val Asp Val Ser His
           275                 280                 285
   Glu Asp Pro Glu Val Lys Phe Asn Trp Tyr Val Asp Gly Val Glu Val
       290                 295                 300
   His Asn Ala Lys Thr Lys Pro Arg Glu Glu Gln Tyr Asn Ser Thr Tyr
   305                 310                 315                 320
   Arg Val Val Ser Val Leu Thr Val Leu His Gln Asp Trp Leu Asn Gly
                   325                 330                 335
   Lys Glu Tyr Lys Cys Lys Val Ser Asn Lys Ala Leu Pro Ala Pro Ile
               340                 345                 350
   Glu Lys Thr Ile Ser Lys Ala Lys Gly Gln Pro Arg Glu Pro Gln Val
           355                 360                 365
   Tyr Thr Leu Pro Pro Ser Arg Asp Glu Leu Thr Lys Asn Gln Val Ser
       370                 375                 380
   Leu Thr Cys Leu Val Lys Gly Phe Tyr Pro Ser Asp Ile Ala Val Glu
   385                 390                 395                 400
   Trp Glu Ser Asn Gly Gln Pro Glu Asn Asn Tyr Lys Thr Thr Pro Pro
                   405                 410                 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
       450                 455                 460
   Pro Gly Lys
   465
<210> 8
   <211> 1404
   <212> DNA
   <213> Homo sapiens
<400> 8
   atggaatgga tctggatctt tctcctcatc ctgtcaggaa ctcgaggtgt ccagtcccag     60
   gttcagctgg tgcagtctgg agctgaagtg aagaagcctg gggcttcagt gaaggtgtcc    120
   tgtaaggctt ctggatacac attcactgcc tatgttataa gctgggtgag gcaggcacct    180
   ggacagggcc ttgagtggat gggagagatt tatcctggaa gcggtagtag ttattataat    240
   gagaagttca agggcagggt cacaatgact agagacacat ccaccagcac agtctacatg    300
   gaactcagca gcctgaggtc tgaggacact gcggtctatt actgtgcaag atccggggac    360
   ggcagtcggt ttgtttactg gggccaaggg acactagtca cagtctcctc agcctccacc    420
   aagggcccat cggtcttccc cctggcaccc tcctccaaga gcacctctgg gggcacagcg    480
   gccctgggct gcctggtcaa ggactacttc cccgaaccgg tgacggtgtc gtggaactca    540
   ggcgccctga ccagcggcgt gcacaccttc ccggctgtcc tacagtcctc aggactctac    600
   tccctcagca gcgtggtgac cgtgccctcc agcagcttgg gcacccagac ctacatctgc    660
   aacgtgaatc acaagcccag caacaccaag gtggacaaga aagttgagcc caaatcttgt    720
   gacaaaactc acacatgccc accgtgccca gcacctgaac tcgcgggggc accgtcagtc    780
   ttcctcttcc ccccaaaacc caaggacacc ctcatgatct cccggacccc tgaggtcaca    840
   tgcgtggtgg tggacgtgag ccacgaagac cctgaggtca agttcaactg gtacgtggac    900
   ggcgtggagg tgcataatgc caagacaaag ccgcgggagg agcagtacaa cagcacgtac    960
   cgtgtggtca gcgtcctcac cgtcctgcac caggactggc tgaatggcaa ggagtacaag   1020
   tgcaaggtct ccaacaaagc cctcccagcc cccatcgaga aaaccatctc caaagccaaa   1080
   gggcagcccc gagaaccaca ggtgtacacc ctgcccccat cccgggatga gctgaccaag   1140
   aaccaggtca gcctgacctg cctggtcaaa ggcttctatc ccagcgacat cgccgtggag   1200
   tgggagagca atgggcagcc ggagaacaac tacaagacca cgcctcccgt gctggactcc   1260
   gacggctcct tcttcctcta cagcaagctc accgtggaca agagcaggtg gcagcagggg   1320
   aacgtcttct catgctccgt gatgcatgag gctctgcaca accactacac gcagaagagc   1380
   ctctccctgt ctccgggtaa atga                                          1404
<210> 9
   <211> 467
   <212> PRT
   <213> Homo sapiens
<400> 9
   Met Glu Trp Ile Trp Ile Phe Leu Leu Ile Leu Ser Gly Thr Arg Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
   35 40 45
   Thr Ala Tyr Val Ile Ser Trp Val Arg Gln Ala Pro Gly Gln Gly Leu
   50 55 60
   Glu Trp Met Gly Glu Ile Tyr Pro Gly Ser Gly Ser Ser Tyr Tyr Asn
   65 70 75 80
   Glu Lys Phe Lys Gly Arg Val Thr Met Thr Arg Asp Thr Ser Thr Ser
   85 90 95
   Thr Val Tyr Met Glu Leu Ser Ser Leu Arg Ser Glu Asp Thr Ala Val
   100 105 110
   Tyr Tyr Cys Ala Arg Ser Gly Asp Gly Ser Arg Phe Val Tyr Trp Gly
   115 120 125
   Gln Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Ala Gly
   245 250 255
   Ala Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Asn Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Asp Glu Leu Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
   420 425 430
   Asp Lys Ser Arg Trp Gln Gln Gly Asn Val Phe Ser Cys Ser Val Met
   435 440 445
   His Glu Ala Leu His Asn His Tyr Thr Gln Lys Ser Leu Ser Leu Ser
   450 455 460
   Pro Gly Lys
   465
<210> 10
   <211> 448
   <212> PRT
   <213> Homo sapiens
<400> 10
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ala Tyr
   20 25 30
   Val Ile Ser Trp Val Arg Gln Ala Pro Gly Gln Gly Leu Glu Trp Met
   35 40 45
   Gly Glu Ile Tyr Pro Gly Ser Gly Ser Ser Tyr Tyr Asn Glu Lys Phe
   50 55 60
   Lys Gly Arg Val Thr Met Thr Arg Asp Thr Ser Thr Ser Thr Val Tyr
   65 70 75 80
   Met Glu Leu Ser Ser Leu Arg Ser Glu Asp Thr Ala Val Tyr Tyr Cys
   85 90 95
   Ala Arg Ser Gly Asp Gly Ser Arg Phe Val Tyr Trp Gly Gln Gly Thr
   100 105 110
   Leu Val Thr Val Ser Ser Ala Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Leu Ala Gly Ala Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Leu Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 11
   <211> 717
   <212> DNA
   <213> Homo sapiens
<400> 11
   atggagacag acacaatcct gctatgggtg ctgctgctct gggttccagg ctccactggt     60
   gacattgtga tgacccaatc tccagattct ttggctgtgt ctctaggtga gagggccacc    120
   atcaactgca aggccagcca aagtgttgat tatgatggtg atagttatat gaactggtat    180
   caacagaaac caggacagcc acccaaactc ctcatctatg ttgcatccaa tctagagtct    240
   ggggtcccag acaggtttag tggcagtggg tctgggacag acttcaccct caccatcagt    300
   tctctgcagg cggaggatgt tgcagtctat tactgtcagc aaagtcttca ggaccctccg    360
   acgttcggtg gaggtaccaa ggtggaaatc aaacgaactg tggctgcact atctgtcttc    420
   atcttcccgc catctgatga gcagttgaaa tctggaactg cctctgttgt gtgcctgctg    480
   aataacttct atcccagaga ggccaaagta cagtggaagg tggataacgc cctccaatcg    540
   ggtaactccc aggagagtgt cacagagcag gacagcaagg acagcaccta cagcctcagc    600
   agcaccctga cgctgagcaa agcagactac gagaaacaca aagtctacgc ctgcgaagtc    660
   acccatcagg gcctgagctc gcccgtcaca aagagcttca acaggggaga gtgttag       717
<210> 12
   <211> 238
   <212> PRT
   <213> Homo sapiens
<400> 12
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65                  70                  75                  80
   Gly Val Pro Asp Arg Phe Ser Gly Ser Gly Ser Gly Thr Asp Phe Thr
                   85                  90                  95
   Leu Thr Ile Ser Ser Leu Gln Ala Glu Asp Val Ala Val Tyr Tyr Cys
               100                 105                 110
   Gln Gln Ser Leu Gln Asp Pro Pro Thr Phe Gly Gly Gly Thr Lys Val
           115                 120                 125
   Glu Ile Lys Arg Thr Val Ala Ala Leu Ser Val Phe Ile Phe Pro Pro
       130                 135                 140
   Ser Asp Glu Gln Leu Lys Ser Gly Thr Ala Ser Val Val Cys Leu Leu
   145                 150                 155                 160
   Asn Asn Phe Tyr Pro Arg Glu Ala Lys Val Gln Trp Lys Val Asp Asn
                   165                 170                 175
   Ala Leu Gln Ser Gly Asn Ser Gln Glu Ser Val Thr Glu Gln Asp Ser
               180                 185                 190
   Lys Asp Ser Thr Tyr Ser Leu Ser Ser Thr Leu Thr Leu Ser Lys Ala
           195                 200                 205
   Asp Tyr Glu Lys His Lys Val Tyr Ala Cys Glu Val Thr His Gln Gly
       210                 215                 220
   Leu Ser Ser Pro Val Thr Lys Ser Phe Asn Arg Gly Glu Cys
   225                 230                 235
<210> 13
   <211> 717
   <212> DNA
   <213> Homo sapiens
<400> 13
   atggagacag acacaatcct gctatgggtg ctgctgctct gggttccagg ctccactggt     60
   gacattgtga tgacccaatc tccagattct ttggctgtgt ctctaggtga gagggccacc    120
   atcaactgca aggccagcca aagtgttgat tatgatggtg atagttatat gaactggtat    180
   caacagaaac caggacagcc acccaaactc ctcatctatg ttgcatccaa tctagagtct    240
   ggggtcccag acaggtttag tggcagtggg tctgggacag acttcaccct caccatcagt    300
   tctctgcagg cggaggatgt tgcagtctat tactgtcagc aaagtcttca ggaccctccg    360
   acgttcggtg gaggtaccaa ggtggaaatc aaacgaactg tggctgcact atctgtcttc    420
   atcttcccgc catctgatga gcagttgaaa tctggaactg cctctgttgt gtgcctgctg    480
   aataacttct atcccagaga ggccaaagta cagtggaagg tggataacgc cctccaatcg    540
   ggtaactccc aggagagtgt cacagagcag gacagcaagg acagcaccta cagcctcagc    600
   agcaccctga cgctgagcaa agcagactac gagaaacaca aagtctacgc ctgcgaagtc    660
   acccatcagg gcctgagctc gcccgtcaca aagagcttca acaggggaga gtgttag       717
<210> 14
   <211> 238
   <212> PRT
   <213> Homo sapiens
<400> 14
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65 70 75 80
   Gly Val Pro Asp Arg Phe Ser Gly Ser Gly Ser Gly Thr Asp Phe Thr
   85 90 95
   Leu Thr Ile Ser Ser Leu Gln Ala Glu Asp Val Ala Val Tyr Tyr Cys
   100 105 110
   Gln Gln Ser Leu Gln Asp Pro Pro Thr Phe Gly Gly Gly Thr Lys Val
   115 120 125
   Glu Ile Lys Arg Thr Val Ala Ala Leu Ser Val Phe Ile Phe Pro Pro
   130 135 140
   Ser Asp Glu Gln Leu Lys Ser Gly Thr Ala Ser Val Val Cys Leu Leu
   145 150 155 160
   Asn Asn Phe Tyr Pro Arg Glu Ala Lys Val Gln Trp Lys Val Asp Asn
   165 170 175
   Ala Leu Gln Ser Gly Asn Ser Gln Glu Ser Val Thr Glu Gln Asp Ser
   180 185 190
   Lys Asp Ser Thr Tyr Ser Leu Ser Ser Thr Leu Thr Leu Ser Lys Ala
   195 200 205
   Asp Tyr Glu Lys His Lys Val Tyr Ala Cys Glu Val Thr His Gln Gly
   210 215 220
   Leu Ser Ser Pro Val Thr Lys Ser Phe Asn Arg Gly Glu Cys
   225 230 235
<210> 15
   <211> 218
   <212> PRT
   <213> Homo sapiens
<400> 15
   Asp Ile Val Met Thr Gln Ser Pro Asp Ser Leu Ala Val Ser Leu Gly
   1 5 10 15
   Glu Arg Ala Thr Ile Asn Cys Lys Ala Ser Gln Ser Val Asp Tyr Asp
   20 25 30
   Gly Asp Ser Tyr Met Asn Trp Tyr Gln Gln Lys Pro Gly Gln Pro Pro
   35 40 45
   Lys Leu Leu Ile Tyr Val Ala Ser Asn Leu Glu Ser Gly Val Pro Asp
       50                  55                  60
   Arg Phe Ser Gly Ser Gly Ser Gly Thr Asp Phe Thr Leu Thr Ile Ser
   65                  70                  75                  80
   Ser Leu Gln Ala Glu Asp Val Ala Val Tyr Tyr Cys Gln Gln Ser Leu
                   85                  90                  95
   Gln Asp Pro Pro Thr Phe Gly Gly Gly Thr Lys Val Glu Ile Lys Arg
               100                 105                 110
   Thr Val Ala Ala Leu Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 16
   <211> 1404
   <212> DNA
   <213> Homo sapiens
<400> 16
   atggaatgga tctggatctt tctcctcatc ctgtcaggaa ctcgaggtgt ccagtcccag      60
   gttcagctgg tgcagtctgg agctgaagtg aagaagcctg gggcttcagt gaaggtgtcc     120
   tgtaaggctt ctggatacac attcactgcc tatgttataa gctgggtgag gcaggcacct     180
   ggacagggcc ttgagtggat gggagagatt tatcctggaa gcggtagtag ttattataat     240
   gagaagttca agggcagggt cacaatgact agagacacat ccaccagcac agtctacatg     300
   gaactcagca gcctgaggtc tgaggacact gcggtctatt actgtgcaag atccggggac     360
   ggcagtcggt ttgtttactg gggccaaggg acactagtca cagtctcctc agcctccacc     420
   aagggcccat cggtcttccc cctggcaccc tcctccaaga gcacctctgg gggcacagcg     480
   gccctgggct gcctggtcaa ggactacttc cccgaaccgg tgacggtgtc gtggaactca     540
   ggcgccctga ccagcggcgt gcacaccttc ccggctgtcc tacagtcctc aggactctac     600
   tccctcagca gcgtggtgac cgtgccctcc agcagcttgg gcacccagac ctacatctgc     660
   aacgtgaatc acaagcccag caacaccaag gtggacaaga aagttgagcc caaatcttgt     720
   gacaaaactc acacatgccc accgtgccca gcacctgaac tcctgggggg accgtcagtc     780
   ttcctcttcc ccccaaaacc caaggacacc ctcatgatct cccggacccc tgaggtcaca     840
   tgcgtggtgg tggacgtgag ccacgaagac cctgaggtca agttcaactg gtacgtggac     900
   ggcgtggagg tgcataatgc caagacaaag ccgcgggagg agcagtacgc cagcacgtac     960
   cgtgtggtca gcgtcctcac cgtcctgcac caggactggc tgaatggcaa ggagtacaag    1020
   tgcaaggtct ccaacaaagc cctcccagcc cccatcgaga aaaccatctc caaagccaaa    1080
   gggcagcccc gagaaccaca ggtgtacacc ctgcccccat cccgggatga gctgaccaag    1140
   aaccaggtca gcctgacctg cctggtcaaa ggcttctatc ccagcgacat cgccgtggag    1200
   tgggagagca atgggcagcc ggagaacaac tacaagacca cgcctcccgt gctggactcc    1260
   gacggctcct tcttcctcta cagcaagctc accgtggaca agagcaggtg gcagcagggg    1320
   aacgtcttct catgctccgt gatgcatgag gctctgcaca accactacac gcagaagagc    1380
   ctctccctgt ctccgggtaa atga                                            1404
<210> 17
   <211> 467
   <212> PRT
   <213> Homo sapiens
<400> 17
   Met Glu Trp Ile Trp Ile Phe Leu Leu Ile Leu Ser Gly Thr Arg Gly
   1 5 10 15
   Val Gln Ser Gln Val Gln Leu Val Gln Ser Gly Ala Glu Val Lys Lys
   20 25 30
   Pro Gly Ala Ser Val Lys Val Ser Cys Lys Ala Ser Gly Tyr Thr Phe
   35 40 45
   Thr Ala Tyr Val Ile Ser Trp Val Arg Gln Ala Pro Gly Gln Gly Leu
   50 55 60
   Glu Trp Met Gly Glu Ile Tyr Pro Gly Ser Gly Ser Ser Tyr Tyr Asn
   65 70 75 80
   Glu Lys Phe Lys Gly Arg Val Thr Met Thr Arg Asp Thr Ser Thr Ser
                   85                  90                  95
   Thr Val Tyr Met Glu Leu Ser Ser Leu Arg Ser Glu Asp Thr Ala Val
               100                 105                 110
   Tyr Tyr Cys Ala Arg Ser Gly Asp Gly Ser Arg Phe Val Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Ser Leu Gly Thr Gln Thr Tyr Ile Cys Asn Val Asn His
       210                 215                 220
   Lys Pro Ser Asn Thr Lys Val Asp Lys Lys Val Glu Pro Lys Ser Cys
   225                 230                 235                 240
   Asp Lys Thr His Thr Cys Pro Pro Cys Pro Ala Pro Glu Leu Leu Gly
                   245                 250                 255
   Gly Pro Ser Val Phe Leu Phe Pro Pro Lys Pro Lys Asp Thr Leu Met
               260                 265                 270
   Ile Ser Arg Thr Pro Glu Val Thr Cys Val Val Val Asp Val Ser His
           275                 280                 285
   Glu Asp Pro Glu Val Lys Phe Asn Trp Tyr Val Asp Gly Val Glu Val
       290                 295                 300
   His Asn Ala Lys Thr Lys Pro Arg Glu Glu Gln Tyr Ala Ser Thr Tyr
   305                 310                 315                 320
   Arg Val Val Ser Val Leu Thr Val Leu His Gln Asp Trp Leu Asn Gly
                   325                 330                 335
   Lys Glu Tyr Lys Cys Lys Val Ser Asn Lys Ala Leu Pro Ala Pro Ile
               340                 345                 350
   Glu Lys Thr Ile Ser Lys Ala Lys Gly Gln Pro Arg Glu Pro Gln Val
           355                 360                 365
   Tyr Thr Leu Pro Pro Ser Arg Asp Glu Leu Thr Lys Asn Gln Val Ser
       370                 375                 380
   Leu Thr Cys Leu Val Lys Gly Phe Tyr Pro Ser Asp Ile Ala Val Glu
   385                 390                 395                 400
   Trp Glu Ser Asn Gly Gln Pro Glu Asn Asn Tyr Lys Thr Thr Pro Pro
                   405                 410                 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
       450                 455                 460
   Pro Gly Lys
   465
<210> 18
   <211> 1404
   <212> DNA
   <213> Homo sapiens
<400> 18
   atggaatgga tctggatctt tctcctcatc ctgtcaggaa ctcgaggtgt ccagtcccag    60
   gttcagctgg tgcagtctgg agctgaagtg aagaagcctg gggcttcagt gaaggtgtcc   120
   tgtaaggctt ctggatacac attcactgcc tatgttataa gctgggtgag gcaggcacct   180
   ggacagggcc ttgagtggat gggagagatt tatcctggaa gcggtagtag ttattataat   240
   gagaagttca agggcagggt cacaatgact agagacacat ccaccagcac agtctacatg   300
   gaactcagca gcctgaggtc tgaggacact gcggtctatt actgtgcaag atccggggac   360
   ggcagtcggt ttgtttactg gggccaaggg acactagtca cagtctcctc agcctccacc   420
   aagggcccat cggtcttccc cctggcaccc tcctccaaga gcacctctgg gggcacagcg   480
   gccctgggct gcctggtcaa ggactacttc cccgaaccgg tgacggtgtc gtggaactca   540
   ggcgccctga ccagcggcgt gcacaccttc ccggctgtcc tacagtcctc aggactctac   600
   tccctcagca gcgtggtgac cgtgccctcc agcagcttgg gcacccagac ctacatctgc   660
   aacgtgaatc acaagcccag caacaccaag gtggacaaga aagttgagcc caaatcttgt   720
   gacaaaactc acacatgccc accgtgccca gcacctgaac tcctgggggg accgtcagtc     780
   ttcctcttcc ccccaaaacc caaggacacc ctcatgatct cccggacccc tgaggtcaca     840
   tgcgtggtgg tggacgtgag ccacgaagac cctgaggtca agttcaactg gtacgtggac     900
   ggcgtggagg tgcataatgc caagacaaag ccgcgggagg agcagtacgc cagcacgtac     960
   cgtgtggtca gcgtcctcac cgtcctgcac caggactggc tgaatggcaa ggagtacaag    1020
   tgcaaggtct ccaacaaagc cctcccagcc cccatcgaga aaaccatctc caaagccaaa    1080
   gggcagcccc gagaaccaca ggtgtacacc ctgcccccat cccgggatga gctgaccaag    1140
   aaccaggtca gcctgacctg cctggtcaaa ggcttctatc ccagcgacat cgccgtggag    1200
   tgggagagca atgggcagcc ggagaacaac tacaagacca cgcctcccgt gctggactcc    1260
   gacggctcct tcttcctcta cagcaagctc accgtggaca agagcaggtg gcagcagggg    1320
   aacgtcttct catgctccgt gatgcatgag gctctgcaca accactacac gcagaagagc    1380
   ctctccctgt ctccgggtaa atga                                           1404
<210> 19
   <211> 467
   <212> PRT
   <213> Homo sapiens
<400> 19
   Met Glu Trp Ile Trp Ile Phe Leu Leu Ile Leu Ser Gly Thr Arg Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Ala Tyr Val Ile Ser Trp Val Arg Gln Ala Pro Gly Gln Gly Leu
       50                  55                  60
   Glu Trp Met Gly Glu Ile Tyr Pro Gly Ser Gly Ser Ser Tyr Tyr Asn
   65                  70                  75                  80
   Glu Lys Phe Lys Gly Arg Val Thr Met Thr Arg Asp Thr Ser Thr Ser
                   85                  90                  95
   Thr Val Tyr Met Glu Leu Ser Ser Leu Arg Ser Glu Asp Thr Ala Val
               100                 105                 110
   Tyr Tyr Cys Ala Arg Ser Gly Asp Gly Ser Arg Phe Val Tyr Trp Gly
           115                 120                 125
   Gln Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Leu Gly
   245 250 255
   Gly Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Ala Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Asp Glu Leu Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
   420 425 430
   Asp Lys Ser Arg Trp Gln Gln Gly Asn Val Phe Ser Cys Ser Val Met
   435 440 445
   His Glu Ala Leu His Asn His Tyr Thr Gln Lys Ser Leu Ser Leu Ser
   450 455 460
   Pro Gly Lys
   465
<210> 20
   <211> 448
   <213> PRT
   <213> Homo sapiens
<400> 20
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ala Tyr
   20 25 30
   Val Ile Ser Trp Val Arg Gln Ala Pro Gly Gln Gly Leu Glu Trp Met
   35 40 45
   Gly Glu Ile Tyr Pro Gly Ser Gly Ser Ser Tyr Tyr Asn Glu Lys Phe
   50 55 60
   Lys Gly Arg Val Thr Met Thr Arg Asp Thr Ser Thr Ser Thr Val Tyr
   65 70 75 80
   Met Glu Leu Ser Ser Leu Arg Ser Glu Asp Thr Ala Val Tyr Tyr Cys
   85 90 95
   Ala Arg Ser Gly Asp Gly Ser Arg Phe Val Tyr Trp Gly Gln Gly Thr
   100 105 110
   Leu Val Thr Val Ser Ser Ala Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
   180 185 190
   Ser Leu Gly Thr Gln Thr Tyr Ile Cys Asn Val Asn His Lys Pro Ser
   195 200 205
   Asn Thr Lys Val Asp Lys Lys Val Glu Pro Lys Ser Cys Asp Lys Thr
   210 215 220
   His Thr Cys Pro Pro Cys Pro Ala Pro Glu Leu Leu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Ala Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Asp Glu Leu Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 21
   <211> 717
   <222> DNA
   <213> Homo sapiens
<400> 21
   atggagacag acacaatcct gctatgggtg ctgctgctct gggttccagg ctccactggt 60
   gacattgtga tgacccaatc tccagattct ttggctgtgt ctctaggtga gagggccacc 120
   atcaactgca aggccagcca aagtgttgat tatgatggtg atagttatat gaactggtat 180
   caacagaaac caggacagcc acccaaactc ctcatctatg ttgcatccaa tctagagtct 240
   ggggtcccag acaggtttag tggcagtggg tctgggacag acttcaccct caccatcagt 300
   tctctgcagg cggaggatgt tgcagtctat tactgtcagc aaagtcttca ggaccctccg 360
   acgttcggtg gaggtaccaa ggtggaaatc aaacgaactg tggctgcact atctgtcttc 420
   atcttcccgc catctgatga gcagttgaaa tctggaactg cctctgttgt gtgcctgctg 480
   aataacttct atcccagaga ggccaaagta cagtggaagg tggataacgc cctccaatcg 540
   ggtaactccc aggagagtgt cacagagcag gacagcaagg acagcaccta cagcctcagc 600
   agcaccctga cgctgagcaa agcagactac gagaaacaca aagtctacgc ctgcgaagtc 660
   acccatcagg gcctgagctc gcccgtcaca aagagcttca acaggggaga gtgttag 717
<210> 22
   <211> 238
   <212> PRT
   <213> Homo sapiens
<400> 22
   Met Glu Thr Asp Thr Ile Leu Leu Trp Val Leu Leu Leu Trp Val Pro
   1 5 10 15
   Gly Ser Thr Gly Asp Ile Val Met Thr Gln Ser Pro Asp Ser Leu Ala
   20 25 30
   Val Ser Leu Gly Glu Arg Ala Thr Ile Asn Cys Lys Ala Ser Gln Ser
   35 40 45
   Val Asp Tyr Asp Gly Asp Ser Tyr Met Asn Trp Tyr Gln Gln Lys Pro
   50 55 60
   Gly Gln Pro Pro Lys Leu Leu Ile Tyr Val Ala Ser Asn Leu Glu Ser
   65 70 75 80
   Gly Val Pro Asp Arg Phe Ser Gly Ser Gly Ser Gly Thr Asp Phe Thr
   85 90 95
   Leu Thr Ile Ser Ser Leu Gln Ala Glu Asp Val Ala Val Tyr Tyr Cys
   100 105 110
   Gln Gln Ser Leu Gln Asp Pro Pro Thr Phe Gly Gly Gly Thr Lys Val
   115 120 125
   Glu Ile Lys Arg Thr Val Ala Ala Leu Ser Val Phe Ile Phe Pro Pro
   130 135 140
   Ser Asp Glu Gln Leu Lys Ser Gly Thr Ala Ser Val Val Cys Leu Leu
   145 150 155 160
   Asn Asn Phe Tyr Pro Arg Glu Ala Lys Val Gln Trp Lys Val Asp Asn
   165 170 175
   Ala Leu Gln Ser Gly Asn Ser Gln Glu Ser Val Thr Glu Gln Asp Ser
   180 185 190
   Lys Asp Ser Thr Tyr Ser Leu Ser Ser Thr Leu Thr Leu Ser Lys Ala
   195 200 205
   Asp Tyr Glu Lys His Lys Val Tyr Ala Cys Glu Val Thr His Gln Gly
   210 215 220
   Leu Ser Ser Pro Val Thr Lys Ser Phe Asn Arg Gly Glu Cys
   225 230 235
<210> 23
   <211> 717
   <212> DNA
   <213> Homo sapiens
<400> 23
   atggagacag acacaatcct gctatgggtg ctgctgctct gggttccagg ctccactggt 60
   gacattgtga tgacccaatc tccagattct ttggctgtgt ctctaggtga gagggccacc 120
   atcaactgca aggccagcca aagtgttgat tatgatggtg atagttatat gaactggtat 180
   caacagaaac caggacagcc acccaaactc ctcatctatg ttgcatccaa tctagagtct 240
   ggggtcccag acaggtttag tggcagtggg tctgggacag acttcaccct caccatcagt     300
   tctctgcagg cggaggatgt tgcagtctat tactgtcagc aaagtcttca ggaccctccg     360
   acgttcggtg gaggtaccaa ggtggaaatc aaacgaactg tggctgcact atctgtcttc     420
   atcttcccgc catctgatga gcagttgaaa tctggaactg cctctgttgt gtgcctgctg     480
   aataacttct atcccagaga ggccaaagta cagtggaagg tggataacgc cctccaatcg     540
   ggtaactccc aggagagtgt cacagagcag gacagcaagg acagcaccta cagcctcagc     600
   agcaccctga cgctgagcaa agcagactac gagaaacaca aagtctacgc ctgcgaagtc     660
   acccatcagg gcctgagctc gcccgtcaca aagagcttca acaggggaga gtgttag        717
<210> 24
   <211> 238
   <212> PRT
   <213> Homo sapiens
<400> 24
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65                  70                  75                  80
   Gly Val Pro Asp Arg Phe Ser Gly Ser Gly Ser Gly Thr Asp Phe Thr
                   85                  90                  95
   Leu Thr Ile Ser Ser Leu Gln Ala Glu Asp Val Ala Val Tyr Tyr Cys
               100                 105                 110
   Gln Gln Ser Leu Gln Asp Pro Pro Thr Phe Gly Gly Gly Thr Lys Val
           115                 120                 125
   Glu Ile Lys Arg Thr Val Ala Ala Leu Ser Val Phe Ile Phe Pro Pro
       130                 135                 140
   Ser Asp Glu Gln Leu Lys Ser Gly Thr Ala Ser Val Val Cys Leu Leu
   145                 150                 155                 160
   Asn Asn Phe Tyr Pro Arg Glu Ala Lys Val Gln Trp Lys Val Asp Asn
   165 170 175
   Ala Leu Gln Ser Gly Asn Ser Gln Glu Ser Val Thr Glu Gln Asp Ser
   180 185 190
   Lys Asp Ser Thr Tyr Ser Leu Ser Ser Thr Leu Thr Leu Ser Lys Ala
   195 200 205
   Asp Tyr Glu Lys His Lys Val Tyr Ala Cys Glu Val Thr His Gln Gly
   210 215 220
   Leu Ser Ser Pro Val Thr Lys Ser Phe Asn Arg Gly Glu Cys
   225 230 235
<210> 25
   <211> 218
   <212> PRT
   <213> Homo sapiens
<400> 25
   Asp Ile Val Met Thr Gln Ser Pro Asp Ser Leu Ala Val Ser Leu Gly
   1 5 10 15
   Glu Arg Ala Thr Ile Asn Cys Lys Ala Ser Gln Ser Val Asp Tyr Asp
   20 25 30
   Gly Asp Ser Tyr Met Asn Trp Tyr Gln Gln Lys Pro Gly Gln Pro Pro
   35 40 45
   Lys Leu Leu Ile Tyr Val Ala Ser Asn Leu Glu Ser Gly Val Pro Asp
   50 55 60
   Arg Phe Ser Gly Ser Gly Ser Gly Thr Asp Phe Thr Leu Thr Ile Ser
   65 70 75 80
   Ser Leu Gln Ala Glu Asp Val Ala Val Tyr Tyr Cys Gln Gln Ser Leu
   85 90 95
   Gln Asp Pro Pro Thr Phe Gly Gly Gly Thr Lys Val Glu Ile Lys Arg
   100 105 110
   Thr Val Ala Ala Leu Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 26
   <211> 1404
   <212> DNA
   <213> Homo sapiens
<400> 26
   atggaatgga tctggatctt tctcctcatc ctgtcaggaa ctcgaggtgt ccagtcccag 60
   gttcagctgg tgcagtctgg agctgaagtg aagaagcctg gggcttcagt gaaggtgtcc 120
   tgtaaggctt ctggatacac attcactgcc tatgttataa gctgggtgag gcaggcacct 180
   ggacagggcc ttgagtggat gggagagatt tatcctggaa gcggtagtag ttattataat 240
   gagaagttca agggcagggt cacaatgact agagacacat ccaccagcac agtctacatg 300
   gaactcagca gcctgaggtc tgaggacact gcggtctatt actgtgcaag atccggggac 360
   ggcagtcggt ttgtttactg gggccaaggg acactagtca cagtctcctc agcctccacc 420
   aagggcccat cggtcttccc cctggcaccc tcctccaaga gcacctctgg gggcacagcg 480
   gccctgggct gcctggtcaa ggactacttc cccgaaccgg tgacggtgtc gtggaactca 540
   ggcgccctga ccagcggcgt gcacaccttc ccggctgtcc tacagtcctc aggactctac 600
   tccctcagca gcgtggtgac cgtgccctcc agcagcttgg gcacccagac ctacatctgc 660
   aacgtgaatc acaagcccag caacaccaag gtggacaaga aagttgagcc caaatcttgt 720
   gacaaaactc acacatgccc accgtgccca gcacctgaac tcgcgggggc accgtcagtc 780
   ttcctcttcc ccccaaaacc caaggacacc ctcatgatct cccggacccc tgaggtcaca 840
   tgcgtggtgg tggacgtgag ccacgaagac cctgaggtca agttcaactg gtacgtggac 900
   ggcgtggagg tgcataatgc caagacaaag ccgcgggagg agcagtacaa cagcacgtac 960
   cgtgtggtca gcgtcctcac cgtcctgcac caggactggc tgaatggcaa ggagtacaag 1020
   tgcaaggtct ccaacaaagc cctcccagcc cccatcgaga aaaccatctc caaagccaaa 1080
   gggcagcccc gagaaccaca ggtgtacacc ctgcccccat cccgggatga gctgaccaag 1140
   aaccaggtca gcctgacctg cctggtcaaa ggcttctatc ccagcgacat cgccgtggag 1200
   tgggagagca atgggcagcc ggagaacaac tacaagacca cgcctcccgt gctggactcc 1260
   gacggctcct tcttcctcta cagcaagctc accgtggaca agagcaggtg gcagcagggg 1320
   aacgtcttct catgctccgt gatgcatgag gctctgcaca accactacac gcagaagagc 1380
   ctctccctgt ctccgggtaa atga 1404
<210> 27
   <211> 467
   <212> PRT
   <213> Homo sapiens
<400> 27
   Met Glu Trp Ile Trp Ile Phe Leu Leu Ile Leu Ser Gly Thr Arg Gly
   1 5 10 15
   Val Gln Ser Gln Val Gln Leu Val Gln Ser Gly Ala Glu Val Lys Lys
   20 25 30
   Pro Gly Ala Ser Val Lys Val Ser Cys Lys Ala Ser Gly Tyr Thr Phe
   35 40 45
   Thr Ala Tyr Val Ile Ser Trp Val Arg Gln Ala Pro Gly Gln Gly Leu
   50 55 60
   Glu Trp Met Gly Glu Ile Tyr Pro Gly Ser Gly Ser Ser Tyr Tyr Asn
   65 70 75 80
   Glu Lys Phe Lys Gly Arg Val Thr Met Thr Arg Asp Thr Ser Thr Ser
   85 90 95
   Thr Val Tyr Met Glu Leu Ser Ser Leu Arg Ser Glu Asp Thr Ala Val
   100 105 110
   Tyr Tyr Cys Ala Arg Ser Gly Asp Gly Ser Arg Phe Val Tyr Trp Gly
   115 120 125
   Gln Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Ser Leu Gly Thr Gln Thr Tyr Ile Cys Asn Val Asn His
       210                 215                 220
   Lys Pro Ser Asn Thr Lys Val Asp Lys Lys Val Glu Pro Lys Ser Cys
   225                 230                 235                 240
   Asp Lys Thr His Thr Cys Pro Pro Cys Pro Ala Pro Glu Leu Ala Gly
                   245                 250                 255
   Ala Pro Ser Val Phe Leu Phe Pro Pro Lys Pro Lys Asp Thr Leu Met
               260                 265                 270
   Ile Ser Arg Thr Pro Glu Val Thr Cys Val Val Val Asp Val Ser His
           275                 280                 285
   Glu Asp Pro Glu Val Lys Phe Asn Trp Tyr Val Asp Gly Val Glu Val
       290                 295                 300
   His Asn Ala Lys Thr Lys Pro Arg Glu Glu Gln Tyr Asn Ser Thr Tyr
   305                 310                 315                 320
   Arg Val Val Ser Val Leu Thr Val Leu His Gln Asp Trp Leu Asn Gly
                   325                 330                 335
   Lys Glu Tyr Lys Cys Lys Val Ser Asn Lys Ala Leu Pro Ala Pro Ile
               340                 345                 350
   Glu Lys Thr Ile Ser Lys Ala Lys Gly Gln Pro Arg Glu Pro Gln Val
           355                 360                 365
   Tyr Thr Leu Pro Pro Ser Arg Asp Glu Leu Thr Lys Asn Gln Val Ser
       370                 375                 380
   Leu Thr Cys Leu Val Lys Gly Phe Tyr Pro Ser Asp Ile Ala Val Glu
   385                 390                 395                 400
   Trp Glu Ser Asn Gly Gln Pro Glu Asn Asn Tyr Lys Thr Thr Pro Pro
                   405                 410                 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
   450 455 460
   Pro Gly Lys
   465
<210> 28
   <211> 1404
   <212> DNA
   <213> Homo sapiens
<400> 28
   atggaatgga tctggatctt tctcctcatc ctgtcaggaa ctcgaggtgt ccagtcccag     60
   gttcagctgg tgcagtctgg agctgaagtg aagaagcctg gggcttcagt gaaggtgtcc    120
   tgtaaggctt ctggatacac attcactgcc tatgttataa gctgggtgag gcaggcacct    180
   ggacagggcc ttgagtggat gggagagatt tatcctggaa gcggtagtag ttattataat    240
   gagaagttca agggcagggt cacaatgact agagacacat ccaccagcac agtctacatg    300
   gaactcagca gcctgaggtc tgaggacact gcggtctatt actgtgcaag atccggggac    360
   ggcagtcggt ttgtttactg gggccaaggg acactagtca cagtctcctc agcctccacc    420
   aagggcccat cggtcttccc cctggcaccc tcctccaaga gcacctctgg gggcacagcg    480
   gccctgggct gcctggtcaa ggactacttc cccgaaccgg tgacggtgtc gtggaactca    540
   ggcgccctga ccagcggcgt gcacaccttc ccggctgtcc tacagtcctc aggactctac    600
   tccctcagca gcgtggtgac cgtgccctcc agcagcttgg gcacccagac ctacatctgc    660
   aacgtgaatc acaagcccag caacaccaag gtggacaaga aagttgagcc caaatcttgt    720
   gacaaaactc acacatgccc accgtgccca gcacctgaac tcgcgggggc accgtcagtc    780
   ttcctcttcc ccccaaaacc caaggacacc ctcatgatct cccggacccc tgaggtcaca    840
   tgcgtggtgg tggacgtgag ccacgaagac cctgaggtca agttcaactg gtacgtggac    900
   ggcgtggagg tgcataatgc caagacaaag ccgcgggagg agcagtacaa cagcacgtac    960
   cgtgtggtca gcgtcctcac cgtcctgcac caggactggc tgaatggcaa ggagtacaag   1020
   tgcaaggtct ccaacaaagc cctcccagcc cccatcgaga aaaccatctc caaagccaaa   1080
   gggcagcccc gagaaccaca ggtgtacacc ctgcccccat cccgggatga gctgaccaag   1140
   aaccaggtca gcctgacctg cctggtcaaa ggcttctatc ccagcgacat cgccgtggag   1200
   tgggagagca atgggcagcc ggagaacaac tacaagacca cgcctcccgt gctggactcc   1260
   gacggctcct tcttcctcta cagcaagctc accgtggaca agagcaggtg gcagcagggg   1320
   aacgtcttct catgctccgt gatgcatgag gctctgcaca accactacac gcagaagagc   1380
   ctctccctgt ctccgggtaa atga                                          1404
<210> 29
   <211> 467
   <212> PRT
   <213> Homo sapiens
<400> 29
   Met Glu Trp Ile Trp Ile Phe Leu Leu Ile Leu Ser Gly Thr Arg Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Ala Tyr Val Ile Ser Trp Val Arg Gln Ala Pro Gly Gln Gly Leu
       50                  55                  60
   Glu Trp Met Gly Glu Ile Tyr Pro Gly Ser Gly Ser Ser Tyr Tyr Asn
   65                  70                  75                  80
   Glu Lys Phe Lys Gly Arg Val Thr Met Thr Arg Asp Thr Ser Thr Ser
                   85                  90                  95
   Thr Val Tyr Met Glu Leu Ser Ser Leu Arg Ser Glu Asp Thr Ala Val
               100                 105                 110
   Tyr Tyr Cys Ala Arg Ser Gly Asp Gly Ser Arg Phe Val Tyr Trp Gly
           115                 120                 125
   Gln Gly Thr Leu Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys Asp Tyr Phe Pro Glu Pro Val Thr Val
                   165                 170                 175
   Ser Trp Asn Ser Gly Ala Leu Thr Ser Gly Val His Thr Phe Pro Ala
               180                 185                 190
   Val Leu Gln Ser Ser Gly Leu Tyr Ser Leu Ser Ser Val Val Thr Val
           195                 200                 205
   Pro Ser Ser Ser Leu Gly Thr Gln Thr Tyr Ile Cys Asn Val Asn His
       210                 215                 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Ala Gly
   245 250 255
   Ala Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Asn Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Asp Glu Leu Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
   420 425 430
   Asp Lys Ser Arg Trp Gln Gln Gly Asn Val Phe Ser Cys Ser Val Met
   435 440 445
   His Glu Ala Leu His Asn His Tyr Thr Gln Lys Ser Leu Ser Leu Ser
   450 455 460
   Pro Gly Lys
   465
<210> 30
   <211> 448
   <212> PRT
   <213> Homo sapiens
<400> 30
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ala Tyr
               20                  25                  30
   Val Ile Ser Trp Val Arg Gln Ala Pro Gly Gln Gly Leu Glu Trp Met
           35                  40                  45
   Gly Glu Ile Tyr Pro Gly Ser Gly Ser Ser Tyr Tyr Asn Glu Lys Phe
       50                  55                  60
   Lys Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Ser Gly Asp Gly Ser Arg Phe Val Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Ala Gly Ala Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Leu Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 31
   <211> 717
   <212> DNA
   <213> Homo sapiens
<400> 31
   atggagacag acacaatcct gctatgggtg ctgctgctct gggttccagg ctccactggt     60
   gacattgtga tgacccaatc tccagattct ttggctgtgt ctctaggtga gagggccacc    120
   atcaactgca aggccagcca aagtgttgat tatgatggtg atagttatat gaactggtat     180
   caacagaaac caggacagcc acccaaactc ctcatctatg ttgcatccaa tctagagtct     240
   ggggtcccag acaggtttag tggcagtggg tctgggacag acttcaccct caccatcagt     300
   tctctgcagg cggaggatgt tgcagtctat tactgtcagc aaagtcttca ggaccctccg     360
   acgttcggtg gaggtaccaa ggtggaaatc aaacgaactg tggctgcacc atctgtcttc     420
   atcttcccgc catctgatga gcagttgaaa tctggaactg cctctgttgt gtgcctgctg     480
   aataacttct atcccagaga ggccaaagta cagtggaagg tggataacgc cctccaatcg     540
   ggtaactccc aggagagtgt cacagagcag gacagcaagg acagcaccta cagcctcagc     600
   agcaccctga cgctgagcaa agcagactac gagaaacaca aagtctacgc ctgcgaagtc     660
   acccatcagg gcctgagctc gcccgtcaca aagagcttca acaggggaga gtgttag        717
<210> 32
   <211> 238
   <212> PRT
   <213> Homo sapiens
<400> 32
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65                  70                  75                  80
   Gly Val Pro Asp Arg Phe Ser Gly Ser Gly Ser Gly Thr Asp Phe Thr
                   85                  90                  95
   Leu Thr Ile Ser Ser Leu Gln Ala Glu Asp Val Ala Val Tyr Tyr Cys
               100                 105                 110
   Gln Gln Ser Leu Gln Asp Pro Pro Thr Phe Gly Gly Gly Thr Lys Val
           115                 120                 125
   Glu Ile Lys Arg Thr Val Ala Ala Pro Ser Val Phe Ile Phe Pro Pro
       130                 135                 140
   Ser Asp Glu Gln Leu Lys Ser Gly Thr Ala Ser Val Val Cys Leu Leu
   145                 150                 155                 160
   Asn Asn Phe Tyr Pro Arg Glu Ala Lys Val Gln Trp Lys Val Asp Asn
                   165                 170                 175
   Ala Leu Gln Ser Gly Asn Ser Gln Glu Ser Val Thr Glu Gln Asp Ser
               180                 185                 190
   Lys Asp Ser Thr Tyr Ser Leu Ser Ser Thr Leu Thr Leu Ser Lys Ala
           195                 200                 205
   Asp Tyr Glu Lys His Lys Val Tyr Ala Cys Glu Val Thr His Gln Gly
       210                 215                 220
   Leu Ser Ser Pro Val Thr Lys Ser Phe Asn Arg Gly Glu Cys
   225                 230                 235
<210> 33
   <211> 716
   <212> DNA
   <213> Homo sapiens
<400> 33
<210> 34
   <211> 238
   <212> PRT
   <213> Homo sapiens
<400> 34
   Met Glu Thr Asp Thr Ile Leu Leu Trp Val Leu Leu Leu Trp Val Pro
   1               5                   10                  15
   Gly Ser Thr Gly Asp Ile Val Met Thr Gln Ser Pro Asp Ser Leu Ala
               20                  25                  30
   Val Ser Leu Gly Glu Arg Ala Thr Ile Asn Cys Lys Ala Ser Gln Ser
           35                  40                  45
   Val Asp Tyr Asp Gly Asp Ser Tyr Met Asn Trp Tyr Gln Gln Lys Pro
       50                  55                  60
   Gly Gln Pro Pro Lys Leu Leu Ile Tyr Val Ala Ser Asn Leu Glu Ser
   65                  70                  75                  80
   Gly Val Pro Asp Arg Phe Ser Gly Ser Gly Ser Gly Thr Asp Phe Thr
                   85                  90                  95
   Leu Thr Ile Ser Ser Leu Gln Ala Glu Asp Val Ala Val Tyr Tyr Cys
               100                 105                 110
   Gln Gln Ser Leu Gln Asp Pro Pro Thr Phe Gly Gly Gly Thr Lys Val
           115                 120                 125
   Glu Ile Lys Arg Thr Val Ala Ala Pro Ser Val Phe Ile Phe Pro Pro
       130                 135                 140
   Ser Asp Glu Gln Leu Lys Ser Gly Thr Ala Ser Val Val Cys Leu Leu
   145                 150                 155                 160
   Asn Asn Phe Tyr Pro Arg Glu Ala Lys Val Gln Trp Lys Val Asp Asn
                   165                 170                 175
   Ala Leu Gln Ser Gly Asn Ser Gln Glu Ser Val Thr Glu Gln Asp Ser
               180                 185                 190
   Lys Asp Ser Thr Tyr Ser Leu Ser Ser Thr Leu Thr Leu Ser Lys Ala
           195                 200                 205
   Asp Tyr Glu Lys His Lys Val Tyr Ala Cys Glu Val Thr His Gln Gly
       210                 215                 220
   Leu Ser Ser Pro Val Thr Lys Ser Phe Asn Arg Gly Glu Cys
   225                 230                 235
<210> 35
   <211> 218
   <212> PRT
   <313> Homo sapiens
<400> 35
   Asp Ile Val Met Thr Gln Ser Pro Asp Ser Leu Ala Val Ser Leu Gly
   1               5                   10                  15
   Glu Arg Ala Thr Ile Asn Cys Lys Ala Ser Gln Ser Val Asp Tyr Asp
               20                  25                  30
   Gly Asp Ser Tyr Met Asn Trp Tyr Gln Gln Lys Pro Gly Gln Pro Pro
           35                  40                  45
   Lys Leu Leu Ile Tyr Val Ala Ser Asn Leu Glu Ser Gly Val Pro Asp
       50                  55                  60
   Arg Phe Ser Gly Ser Gly Ser Gly Thr Asp Phe Thr Leu Thr Ile Ser
   65                  70                  75                  80
   Ser Leu Gln Ala Glu Asp Val Ala Val Tyr Tyr Cys Gln Gln Ser Leu
                   85                  90                  95
   Gln Asp Pro Pro Thr Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 36
   <211> 1404
   <212> DNA
   <213> Homo sapiens
<400> 36
   atggaatgga tctggatctt tctcctcatc ctgtcaggaa ctcgaggtgt ccagtcccag     60
   gttcagctgg tgcagtctgg agctgaagtg aagaagcctg gggcttcagt gaaggtgtcc    120
   tgtaaggctt ctggatacac attcactgcc tatgttataa gctgggtgag gcaggcacct    180
   ggacagggcc ttgagtggat gggagagatt tatcctggaa gcggtagtag ttattataat    240
   gagaagttca agggcagggt cacaatgact agagacacat ccaccagcac agtctacatg    300
   gaactcagca gcctgaggtc tgaggacact gcggtctatt actgtgcaag atccggggac    360
   ggcagtcggt ttgtttactg gggccaaggg acactagtca cagtctcctc agcctccacc     420
   aagggcccat cggtcttccc cctggcaccc tcctccaaga gcacctctgg gggcacagcg    480
   gccctgggct gcctggtcaa ggactacttc cccgaaccgg tgacggtgtc gtggaactca    540
   ggcgccctga ccagcggcgt gcacaccttc ccggctgtcc tacagtcctc aggactctac    600
   tccctcagca gcgtggtgac cgtgccctcc agcagcttgg gcacccagac ctacatctgc    660
   aacgtgaatc acaagcccag caacaccaag gtggacaaga aagttgagcc caaatcttgt    720
   gacaaaactc acacatgccc accgtgccca gcacctgaac tcctgggggg accgtcagtc    780
   ttcctcttcc ccccaaaacc caaggacacc ctcatgatct cccggacccc tgaggtcaca    840
   tgcgtggtgg tggacgtgag ccacgaagac cctgaggtca agttcaactg gtacgtggac    900
   ggcgtggagg tgcataatgc caagacaaag ccgcgggagg agcagtacgc cagcacgtac    960
   cgtgtggtca gcgtcctcac cgtcctgcac caggactggc tgaatggcaa ggagtacaag   1020
   tgcaaggtct ccaacaaagc cctcccagcc cccatcgaga aaaccatctc caaagccaaa   1080
   gggcagcccc gagaaccaca ggtgtacacc ctgcccccat cccgggatga gctgaccaag   1140
   aaccaggtca gcctgacctg cctggtcaaa ggcttctatc ccagcgacat cgccgtggag   1200
   tgggagagca atgggcagcc ggagaacaac tacaagacca cgcctcccgt gctggactcc   1260
   gacggctcct tcttcctcta cagcaagctc accgtggaca agagcaggtg gcagcagggg   1320
   aacgtcttct catgctccgt gatgcatgag gctctgcaca accactacac gcagaagagc   1380
   ctctccctgt ctccgggtaa atga                                          1404
<210> 37
   <211> 467
   <212> PRT
   <213> Homo sapiens
<400> 37
   Met Glu Trp Ile Trp Ile Phe Leu Leu Ile Leu Ser Gly Thr Arg Gly
   1 5 10 15
   Val Gln Ser Gln Val Gln Leu Val Gln Ser Gly Ala Glu Val Lys Lys
   20 25 30
   Pro Gly Ala Ser Val Lys Val Ser Cys Lys Ala Ser Gly Tyr Thr Phe
   35 40 45
   Thr Ala Tyr Val Ile Ser Trp Val Arg Gln Ala Pro Gly Gln Gly Leu
   50 55 60
   Glu Trp Met Gly Glu Ile Tyr Pro Gly Ser Gly Ser Ser Tyr Tyr Asn
   65 70 75 80
   Glu Lys Phe Lys Gly Arg Val Thr Met Thr Arg Asp Thr Ser Thr Ser
   85 90 95
   Thr Val Tyr Met Glu Leu Ser Ser Leu Arg Ser Glu Asp Thr Ala Val
   100 105 110
   Tyr Tyr Cys Ala Arg Ser Gly Asp Gly Ser Arg Phe Val Tyr Trp Gly
   115 120 125
   Gln Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Leu Gly
   245 250 255
   Gly Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Ala Ser Thr Tyr
   305 310 315 320
   Arg Val Val Ser Val Leu Thr Val Leu His Gln Asp Trp Leu Asn Gly
   325 330 335
   Lys Glu Tyr Lys Cys Lys Val Ser Asn Lys Ala Leu Pro Ala Pro Ile
   340 345 350
   Glu Lys Thr Ile Ser Lys Ala Lys Gly Gln Pro Arg Glu Pro Gln Val
   355 360 365
   Tyr Thr Leu Pro Pro Ser Arg Asp Glu Leu Thr Lys Asn Gln Val Ser
   370 375 380
   Leu Thr Cys Leu Val Lys Gly Phe Tyr Pro Ser Asp Ile Ala Val Glu
   385 390 395 400
   Trp Glu Ser Asn Gly Gln Pro Glu Asn Asn Tyr Lys Thr Thr Pro Pro
   405 410 415
   Val Leu Asp Ser Asp Gly Ser Phe Phe Leu Tyr Ser Lys Leu Thr Val
   420 425 430
   Asp Lys Ser Arg Trp Gln Gln Gly Asn Val Phe Ser Cys Ser Val Met
   435 440 445
   His Glu Ala Leu His Asn His Tyr Thr Gln Lys Ser Leu Ser Leu Ser
   450 455 460
   Pro Gly Lys
   465
<210> 38
   <211> 1404
   <212> DNA
   <213> Homo sapiens
<400> 38
   atggaatgga tctggatctt tctcctcatc ctgtcaggaa ctcgaggtgt ccagtcccag 60
   gttcagctgg tgcagtctgg agctgaagtg aagaagcctg gggcttcagt gaaggtgtcc 120
   tgtaaggctt ctggatacac attcactgcc tatgttataa gctgggtgag gcaggcacct     180
   ggacagggcc ttgagtggat gggagagatt tatcctggaa gcggtagtag ttattataat     240
   gagaagttca agggcagggt cacaatgact agagacacat ccaccagcac agtctacatg     300
   gaactcagca gcctgaggtc tgaggacact gcggtctatt actgtgcaag atccggggac     360
   ggcagtcggt ttgtttactg gggccaaggg acactagtca cagtctcctc agcctccacc     420
   aagggcccat cggtcttccc cctggcaccc tcctccaaga gcacctctgg gggcacagcg     480
   gccctgggct gcctggtcaa ggactacttc cccgaaccgg tgacggtgtc gtggaactca     540
   ggcgccctga ccagcggcgt gcacaccttc ccggctgtcc tacagtcctc aggactctac     600
   tccctcagca gcgtggtgac cgtgccctcc agcagcttgg gcacccagac ctacatctgc     660
   aacgtgaatc acaagcccag caacaccaag gtggacaaga aagttgagcc caaatcttgt     720
   gacaaaactc acacatgccc accgtgccca gcacctgaac tcctgggggg accgtcagtc     780
   ttcctcttcc ccccaaaacc caaggacacc ctcatgatct cccggacccc tgaggtcaca     840
   tgcgtggtgg tggacgtgag ccacgaagac cctgaggtca agttcaactg gtacgtggac     900
   ggcgtggagg tgcataatgc caagacaaag ccgcgggagg agcagtacgc cagcacgtac     960
   cgtgtggtca gcgtcctcac cgtcctgcac caggactggc tgaatggcaa ggagtacaag    1020
   tgcaaggtct ccaacaaagc cctcccagcc cccatcgaga aaaccatctc caaagccaaa    1080
   gggcagcccc gagaaccaca ggtgtacacc ctgcccccat cccgggatga gctgaccaag    1140
   aaccaggtca gcctgacctg cctggtcaaa ggcttctatc ccagcgacat cgccgtggag    1200
   tgggagagca atgggcagcc ggagaacaac tacaagacca cgcctcccgt gctggactcc    1260
   gacggctcct tcttcctcta cagcaagctc accgtggaca agagcaggtg gcagcagggg    1320
   aacgtcttct catgctccgt gatgcatgag gctctgcaca accactacac gcagaagagc    1380
   ctctccctgt ctccgggtaa atga                                            1404
<210> 39
   <211> 467
   <212> PRT
   <213> Homo sapiens
<400> 39
   Met Glu Trp Ile Trp Ile Phe Leu Leu Ile Leu Ser Gly Thr Arg Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Ala Tyr Val Ile Ser Trp Val Arg Gln Ala Pro Gly Gln Gly Leu
   50 55 60
   Glu Trp Met Gly Glu Ile Tyr Pro Gly Ser Gly Ser Ser Tyr Tyr Asn
   65 70 75 80
   Glu Lys Phe Lys Gly Arg Val Thr Met Thr Arg Asp Thr Ser Thr Ser
   85 90 95
   Thr Val Tyr Met Glu Leu Ser Ser Leu Arg Ser Glu Asp Thr Ala Val
   100 105 110
   Tyr Tyr Cys Ala Arg Ser Gly Asp Gly Ser Arg Phe Val Tyr Trp Gly
   115 120 125
   Gln Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys Asp Tyr Phe Pro Glu Pro Val Thr Val
   165 170 175
   Ser Trp Asn Ser Gly Ala Leu Thr Ser Gly Val His Thr Phe Pro Ala
   180 185 190
   Val Leu Gln Ser Ser Gly Leu Tyr Ser Leu Ser Ser Val Val Thr Val
   195 200 205
   Pro Ser Ser Ser Leu Gly Thr Gln Thr Tyr Ile Cys Asn Val Asn His
   210 215 220
   Lys Pro Ser Asn Thr Lys Val Asp Lys Lys Val Glu Pro Lys Ser Cys
   225 230 235 240
   Asp Lys Thr His Thr Cys Pro Pro Cys Pro Ala Pro Glu Leu Leu Gly
   245 250 255
   Gly Pro Ser Val Phe Leu Phe Pro Pro Lys Pro Lys Asp Thr Leu Met
   260 265 270
   Ile Ser Arg Thr Pro Glu Val Thr Cys Val Val Val Asp Val Ser His
   275 280 285
   Glu Asp Pro Glu Val Lys Phe Asn Trp Tyr Val Asp Gly Val Glu Val
   290 295 300
   His Asn Ala Lys Thr Lys Pro Arg Glu Glu Gln Tyr Ala Ser Thr Tyr
   305 310 315 320
   Arg Val Val Ser Val Leu Thr Val Leu His Gln Asp Trp Leu Asn Gly
                   325                 330                 335
   Lys Glu Tyr Lys Cys Lys Val Ser Asn Lys Ala Leu Pro Ala Pro Ile
               340                 345                 350
   Glu Lys Thr Ile Ser Lys Ala Lys Gly Gln Pro Arg Glu Pro Gln Val
           355                 360                 365
   Tyr Thr Leu Pro Pro Ser Arg Asp Glu Leu Thr Lys Asn Gln Val Ser
       370                 375                 380
   Leu Thr Cys Leu Val Lys Gly Phe Tyr Pro Ser Asp Ile Ala Val Glu
   385                 390                 395                 400
   Trp Glu Ser Asn Gly Gln Pro Glu Asn Asn Tyr Lys Thr Thr Pro Pro
                   405                 410                 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
       450                 455                 460
   Pro Gly Lys
   465
<210> 40
   <211> 448
   <212> PRT
   <213> Homo sapiens
<400> 40
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ala Tyr
               20                  25                  30
   Val Ile Ser Trp Val Arg Gln Ala Pro Gly Gln Gly Leu Glu Trp Met
           35                  40                  45
   Gly Glu Ile Tyr Pro Gly Ser Gly Ser Ser Tyr Tyr Asn Glu Lys Phe
       50                  55                  60
   Lys Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Ser Gly Asp Gly Ser Arg Phe Val Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Ala Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
   340 345 350
   Pro Pro Ser Arg Asp Glu Leu Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 41
   <211> 135
   <212> PRT
   <213> Homo sapiens
<400> 41
   Met Val Leu Gln Thr Gln Val Phe Ile Ser Leu Leu Leu Trp Ile Ser
   1 5 10 15
   Gly Ala Tyr Gly Asp Ile Val Met Thr Gln Ser Pro Asp Ser Leu Ala
   20 25 30
   Val Ser Leu Gly Glu Arg Ala Thr Ile Asn Cys Lys Ser Ser Gln Ser
   35 40 45
   Leu Leu Tyr Ser Ser Asn Asn Lys Asn Tyr Leu Ala Trp Tyr Gln Gln
   50 55 60
   Lys Pro Gly Gln Pro Pro Lys Leu Leu Ile Tyr Trp Ala Ser Thr Arg
   65 70 75 80
   Glu Ser Gly Val Pro Asp Arg Phe Ser Gly Ser Gly Ser Gly Thr Asp
   85 90 95
   Phe Thr Leu Thr Ile Ser Ser Leu Gln Ala Glu Asp Val Ala Val Tyr
   100 105 110
   Tyr Cys Gln Gln Tyr Tyr Ser Thr Pro Pro Met Phe Gly Gln Gly Thr
   115 120 125
   Lys Val Glu Ile Lys Arg Thr
   130 135
<210> 42
   <211> 142
   <212> PRT
   <213> Homo sapiens
<400> 42
   Leu Leu Ala Val Ala Pro Gly Ala His Ser Gln Val Gln Leu Val Gln
   1 5 10 15
   Ser Gly Ala Glu Val Lys Lys Pro Gly Ala Ser Val Lys Val Ser Cys
   20 25 30
   Lys Ala Ser Gly Tyr Thr Phe Thr Asn Tyr Tyr Met His Trp Val Arg
   35 40 45
   Gln Ala Pro Gly Gln Gly Leu Glu Trp Met Gly Ile Ile Asn Pro Ser
   50 55 60
   Gly Asn Ser Thr Asn Tyr Ala Gln Lys Phe Gln Gly Arg Val Thr Met
   65 70 75 80
   Thr Arg Asp Thr Ser Thr Ser Thr Val Tyr Met Glu Leu Ser Ser Leu
   85 90 95
   Arg Ser Glu Asp Thr Ala Val Tyr Tyr Cys Ala Arg Glu Lys Leu Ala
   100 105 110
   Thr Thr Ile Phe Gly Val Leu Ile Ile Thr Gly Met Asp Tyr Trp Gly
   115 120 125
   Gln Gly Thr Leu Val Thr Val Ser Ser Gly Ser Ala Ser Ala
   130 135 140
<210> 43
   <211> 76
   <212> DNA
   <213> ARTIFICIAL SEQUENCE
<220>
   <223> PCR Primer
<400> 43
   tgacattgtg atgacccaat ctccagattc tttggctgtg tctctaggtg agagggccac 60
   catcaactgc aaggcc 76
<210> 44
   <211> 29
   <212> DNA
   <213> Artificial sequence
<220>
   <223> PCR Primer
<400> 44
   tgaactggta tcaacagaaa ccaggacag 29
<210> 45
   <211> 28
   <212> DNA
   <213> Artificial sequence
<220>
   <223> PCR Primer
<400> 45
   agagtctggg gtcccagaca ggtttagt 28
<210> 46
   <211> 42
   <212> DNA
   <213> Artificial sequence
<220>
   <223> PCR Primer
<400> 46
   gtcttcagga ccctccgacg ttcggtggag gtaccaagct gg 42
<210> 47
   <211> 52
   <212> DNA
   <213> Artificial sequence
<220>
   <223> PCR Primer
<400> 47
   caccctcacc atcagttctc tgcaggcgga ggatgttgca gtctattagt gt 52
<210> 48
   <211> 24
   <212> DNA
   <213> Artificial sequence
<230>
   <223> PCR Primer
<400> 48
   agctttacag ttactgagca caca 24
<210> 49
   <211> 24
   <212> DNA
   <213> Artificial sequence
<220>
   <223> PCR Primer
<400> 49
   tcgatgtgtg ctcagtaact gtaa 24
<210> 50
   <211> 75
   <212> DNA
   <213> Artificial sequence
<220>
   <223> PCR Primer
<400> 50
   ggttcagctg gtgcagtctg gagctgaagt gaagaagcct ggggcttcag tgaaggtgtc 60
   ctgtaaggct tctgg 75
<210> 51
   <211> 52
   <212> DNA
   <213> Artificial sequence
   <220>
   <223> PCR Primer
<400> 51
   agctgggtga ggcaggcacc tggacagggc cttgagtgga tgggagagat tt 52
<210> 52
   <211> 60
   <212> DNA
   <213> Artificial sequence
<220>
   <223> PCR Primer
<400> 52
   caagggcagg gtcacaatga ctagagacac atccaccagc acagtctaca tggaactcag 60
<210> 53
   <211> 43
   <212> DNA
   <213> Artificial sequence
<220>
   <223> PCR Primer
<400> 53
   cagcctgagg tctgaggaca ctgcggtcta ttactgtgca aga 43
<210> 54
   <211> 24
   <212> DNA
   <213> Artificial sequence
<220>
   <223> PCR Primer
<400> 54
   gccaagggac actagtcact gtgt 24
<210> 55
   <211> 39
   <212> DNA
   <213> Artificial sequence
<220>
   <223> PCR Primer
<400> 55
   tcactgccta tgttataagc tgggtgaggc aggcacctg 39
<210> 56
   <211> 39
   <212> DNA
   <213> Artificial sequence
<220>
   <223> PCR Primer
<400> 56
   actctaacca tggaatggat ctggatcttt ctcctcatc 39
<210> 57
   <211> 21
   <212> DNA
   <213> Artificial sequence
<220>
   <223> PCR Primer
<400> 57
   actagtcaca gtctcctcag c 21
<210> 58
   <211> 22
   <212> DIQA
   <213> Artificial sequence
<220>
   <223> PCR Primer
<400> 58
   gaattcattt acccggagac ag 22
<210> 59
   <211> 49
   <212> DNA
   <213> Artificial sequence
<220>
   <223> PCR Primer
<400> 59
   ccgtgcccag cacctgaact cgcgggggca ccgtcagtct tcctccccc 49
<210> 60
   <211> 26
   <212> DNA
   <213> Artificial sequence
<220>
   <223> PCR Primer
<400> 60
   ggtaccaagg tggaaatcaa acgaac 26
<210> 61
   <211> 25
   <212> DNA
   <213> Artificial sequence
<220>
   <223> PCR Primer
<400> 61
   aagcttctaa cactctcccc tgttg 25
<210> 62
   <211> 78
   <212> DNA
   <213> Artificial sequence
<220>
   <223> PCR Primer
<400> 62
   aagcttatgg aatggatctg gatctttctc ctcatcctgt caggaactcg aggtgtccag 60
   tcccaggttc agctggtg 78
<210> 63
   <211> 74
   <212> DNA
   <213> Artificial sequence
<220>
   <223> PCR Primer
<400> 63
   ctgtaaggct tctggataca cattcactgc ctatgttata agctgggtga ggcaggcacc 60
   tggacagggc cttg 74
<210> 64
   <211> 70
   <212> DNA
   <213> Artificial sequence
<220>
   <223> PCR Primer
<400> 64
   ggtagtagtt attataatga gaagttcaag ggcagggtca caatgactag agacacatcc 60
   accagcacag 70
<210> 65
   <211> 77
   <212> DNA
   <213> Artificial sequence
<220>
   <223> PCR Primer
<400> 65
   gaggacactg cggtctatta ctgtgcaaga tccggggacg gcagtcggtt tgtttactgg 60
   ggccaaggga cactagt 77
<210> 66
   <211> 90
   <212> DNA
   <213> Artificial sequence
<220>
   <223> PCR Primer
<400> 66
   gtgtatccag aagccttaca ggacaccttc actgaagccc caggcttctt cacttcagct 60
   ccagactgca ccagctgaac ctgggactgg 90
<210> 67
   <211> 77
   <212> DNA
   <213> Artificial sequence
<220>
   <223> PCR Primer
<400> 67
   cttctcatta taataactac taccgcttcc aggataaatc tctcccatcc actcaaggcc 60
   ctgtccaggt gcctgcc 77
<210> 68
   <211> 71
   <212> DNA
   <213> Artificial sequence
<230>
   <223> PCR Primer
<400> 68
   gtaatagacc gcagtgtcct cagacctcag gctgctgagt tccatgtaga ctgtgctggt 60
   ggatgtgtct c 71
<210> 69
   <211> 69
   <212> DNA
   <213> Artificial sequence
<220>
   <223> PCR Primer
<400> 69
   gaattcatgg agacagacac aatcctgcta tgggtgctgc tgctctgggt tccaggctcc 60
   actggtgac 69
<210> 70
   <211> 66
   <212> DNA
   <213> Artificial sequence
<220>
   <223> PCR Primer
<400> 70
   ggctgtgtct ctaggtgaga gggccaccat caactgcaag gccagccaaa gtgttgatta 60
   tgatgg 66
<210> 71
   <211> 65
   <212> DNA
   <213> Artificial sequence
<220>
   <223> PCR Primer
<400> 71
   cagaaaccag gacagccacc caaactcctc atctatgttg catccaatct agagtctggg 60
   gtccc 65
<210> 72
   <211> 66
   <212> DNA
   <213> Artificial sequence
<220>
   <223> PCR Primer
<400> 72
   ggacagactt caccctcacc atcagttctc tgcaggcgga ggatgttgca gtctattact 60
   gtcagc 66
<210> 73
   <211> 67
   <212> DNA
   <213> Artificial sequence
<220>
   <223> PCR Primer
<400> 73
   cacctagaga cacagccaaa gaatctggag attgggtcat cacaatgtca ccagtggagc 60
   ctggaac 67
<210> 74
   <211> 66
   <212> DNA
   <213> Artificial sequence
<220>
   <223> PCR Primer
<400> 74
   ggtggctgtc ctggtttctg ttgataccag ttcatataac tatcaccatc ataatcaaca 60
   ctttgg 66
<210> 75
   <211> 67
   <212> DNA
   <213> Artificial sequence
<230>
   <223> PCR Primer
<400> 75
   ggtgagggtg aagtctgtcc cagacccact gccactaaac ctgtctggga ccccagactc 60
   tagattg 67
<210> 76
   <211> 60
   <212> DNA
   <213> Artificial sequence
<220>
   <223> PCR Primer
<400> 76
   ggtacctcca ccgaacgtcg gagggtcctg aagactttgc tgacagtaat agactgcaac 60